(19) Europäisches Patentamt
European Patent Office
Office européen des brevets

(11) EP 4 183 792 A1

(12) **EUROPEAN PATENT APPLICATION**
published in accordance with Art. 153(4) EPC

(43) Date of publication:
**24.05.2023 Bulletin 2023/21**

(21) Application number: **20944871.1**

(22) Date of filing: **14.07.2020**

(51) International Patent Classification (IPC):
***C07H 5/06*** *(2006.01)* ***C07H 1/00*** *(2006.01)*
***C12P 19/26*** *(2006.01)* ***C12P 19/44*** *(2006.01)*
***A61K 31/70*** *(2006.01)* ***A61P 31/00*** *(2006.01)*

(52) Cooperative Patent Classification (CPC):
**A61K 31/70; A61P 31/00; C07H 1/00; C07H 5/06; C12P 19/26; C12P 19/44;** Y02P 20/55

(86) International application number:
**PCT/CN2020/101906**

(87) International publication number:
**WO 2022/011554 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Sichuan University**
  **Chengdu, Sichuan 610065 (CN)**
- **Academy of Military Medical Sciences**
  **Beijing 100850 (CN)**

(72) Inventors:
- **LIU, Xiaoyu**
  **Chengdu, Sichuan 610065 (CN)**
- **QIN, Yong**
  **Chengdu, Sichuan 610065 (CN)**
- **ZHONG, Wu**
  **Beijing 100850 (CN)**
- **CAO, Ruiyuan**
  **Beijing 100850 (CN)**
- **HE, Huan**
  **Chengdu, Sichuan 610065 (CN)**
- **ZHENG, Zhibing**
  **Beijing 100850 (CN)**
- **LI, Song**
  **Beijing 100850 (CN)**

(74) Representative: **Osterhoff, Utz**
**Bockermann Ksoll**
**Griepenstroh Osterhoff**
**Patentanwälte**
**Bergstraße 159**
**44791 Bochum (DE)**

# (54) 3-DEOXY-2-KETONIC ACID NITROGEN-CONTAINING DERIVATIVE, PREPARATION METHOD THEREFOR, AND USE THEREOF

(57) The invention relates to a compound represented by general Formula I, a stereoisomer thereof, a pharmaceutically acceptable salt thereof, a pharmaceutically acceptable ester thereof, a pharmaceutically acceptable hydrate thereof or pharmaceutically acceptable solvate thereof, and the invention also relates to a pharmaceutical composition comprising the compound, and a preparation method for the compound and a use of the compound.

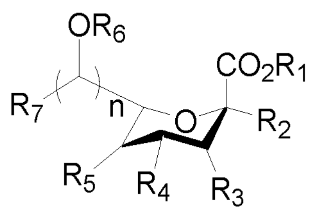

**I** .

EP 4 183 792 A1

## Description

### Technical Field

The present application belongs to the technical field of medicine, and specifically relates to a nitrogen-containing derivative of 3-deoxy-2-ketoaldonic acid, which has antiviral activity. The present application also relates to a pharmaceutical composition comprising the compound, as well as a method for preparing the compound and use of the compound.

### Background Art

3-Deoxy-2-ketoaldonic acid widely exists in nature and plays an important role in the life process. For example, 3-deoxy-D-manno-octulosonic acid (Kdo) is an important component of lipopolysaccharide and capsular polysaccharide of bacterial cell wall. The lipopolysaccharide or capsular polysaccharide containing Kdo fragment are potential antibacterial vaccines or diagnostic agents. N-acetylneuraminic acid is the main member of the sialic acid family, widely present in glycoproteins and glycolipids in animal cell membranes or secretions, and has various biological functions such as anti-inflammatory, anti-viral, anti-tumor, and anti-senile dementia.

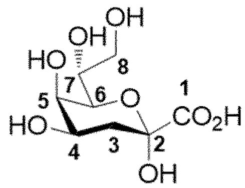

3-deoxy-D-manno-octulosonic acid (Kdo)

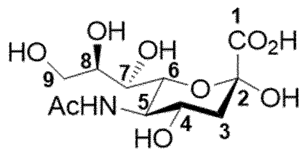

N-acetylneuraminic acid

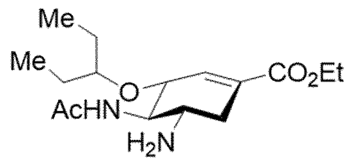

oseltamivir

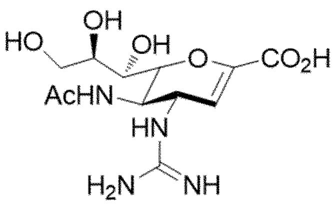

zanamivir

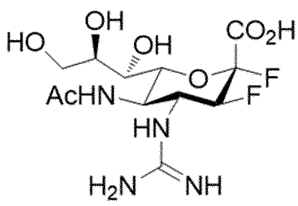

1

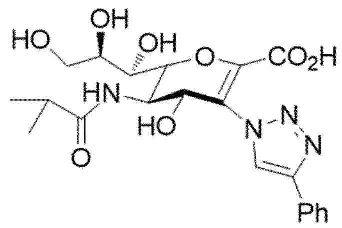

2

Structural modification based on the corresponding 3-deoxy-2-ketoaldonic acid molecule is an important means for the development of new antiviral drugs. For example, oseltamivir and zanamivir are currently antiviral drugs widely used in clinic. the 2,3-difluoro-containing derivative 1 is a broad-spectrum antiviral candidate molecule (Science, 2013, 340, 71-75), and the 3-triazole-substituted derivative 2 is an effective human parainfluenza virus 3 inhibitor (ACS Chem. Biol. 2018, 13, 1544-1550). The preparation of novel derivatives of 3-deoxy-2-ketoaldonic acid by use of efficient synthetic methods has important scientific significance and application value for the discovery of new antiviral drugs.

### Contents of the Application

The purpose of the present application is to find and develop a new type of nitrogen-containing derivatives of 3-deoxy-2-ketoaldonic acid with antiviral activity. The present application has discovered through research that a compound having the structure represented by general Formula I has antiviral activity. The present application has been completed on the basis of the above findings.

The present application relates to a compound represented by general Formula I, a stereoisomer, a pharmaceutically acceptable salt, a pharmaceutically acceptable ester, a pharmaceutically acceptable hydrate or a pharmaceutically acceptable solvate thereof,

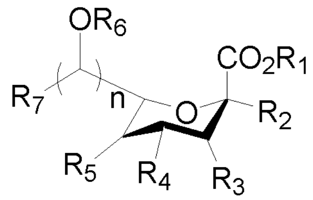

**I**

wherein:

$R_1$ is hydrogen, $C_1$-$C_6$ alkyl, allyl, phenyl or benzyl;

$R_2$ and $R_3$ are each independently azido, amino, amino substituted by one or two $R^a$, 5-membered or6-membered nitrogen-containing heterocyclyl or5-membered or 6-membered nitrogen-containing heterocyclyl substituted by $R^b$, wherein each $R^a$ is independently $C_1$-$C_6$ alkyl, allyl, phenyl, benzyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4;

$R_4$ and $R_5$ are each independently hydroxyl, amino, guanidino, hydroxyl substituted by $R^c$, amino substituted by $R^c$, guanidino substituted by $R^c$, 5-membered or 6-membered nitrogen-containing heterocyclyl or 5-membered or 6-membered nitrogen-containing heterocyclyl substituted by $R^b$, wherein each $R^c$ is independently $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4; or,

$R_4$ and $R_5$ together with the carbon atoms to which they connect form a 5-membered or 6-membered oxygen-containing or nitrogen-containing heterocyclic ring (e.g., 1,3-dioxolane, 1,3-dioxolan-2-one, dioxane, dioxanone, oxazolidine or oxazolidinone);

$R_6$ is hydrogen, $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl;

$R_7$ is hydrogen,

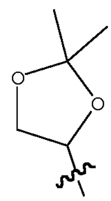

,

$R_6OCH_2$- or $R_6OCH_2(R_6O)CH$-, wherein $R_6$ is as defined above;

n is 0, 1, 2 or 3.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is hydrogen or $C_1$-$C_4$ alkyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ and $R_3$ are each independently azido, amino or 5- or 6-membered nitrogen-containing heterocyclyl substituted by $R^b$, wherein $R^b$ is $-(CH_2)_mOH$, wherein m is 0, 1, 2, 3 or 4, the 5-membered or 6-membered nitrogen-containing heterocyclyl is triazolyl, tetrazolyl, pyrrolyl, tetrahydropyrrolyl, piperidinyl, pyridyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ are each independently hydroxyl, amino,

hydroxyl substituted by $R^c$, amino substituted by $R^c$, wherein each $R^c$ is independently $C_1$-$C_4$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl, methoxyformyl, tert-butoxyformyl or benzyloxyacyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form a 5-membered oxygen-containing heterocyclic ring (e.g., 1,3-dioxolane, 1,3-dioxolan-2-one).

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is formyl, acetyl, benzoyl or trifluoroacetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is

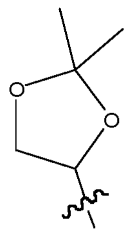

,

$R_6OCH_2$- or $R_6OCH_2(R_6O)CH$-, wherein $R_6$ is as defined above.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: n is 0, 1 or 2.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is hydrogen, methyl, ethyl, n-propyl, isopropyl, n-butyl, isobutyl, sec-butyl or tert-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ and $R_3$ are each independently azido, amino or 5-membered nitrogen-containing heterocyclyl substituted by $R^b$, wherein $R^b$ is $-(CH_2)_mOH$, wherein m is 0, 1, 2 or 3, the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ are each independently hydroxyl, hydroxyl substituted by $R^c$, amino substituted by $R^c$, wherein each $R^c$ is independently methyl, ethyl, n-propyl, isopropyl, n-butyl, isobutyl, sec-butyl, tert-butyl, trimethylsilyl, triethylsilyl, triisopropylsilyl, tert-butyldimethylsilyl, formyl, acetyl, benzoyl, trifluoroacetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is hydrogen or methyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is azido or

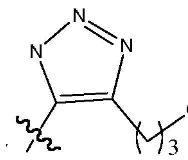

.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$

hydrate or a pharmaceutically acceptable solvate thereof, wherein: $R_3$ is azido, amino or

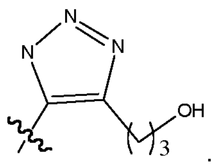

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is hydroxyl, tert-butyldimethylsilyloxy, benzoyloxy, acetylamino or acetyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is hydroxyl, trifluoroacetyloxy, acetylamino or acetyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is acetyl or benzoyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is

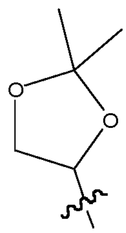

or $R_6OCH_2$-, wherein $R_6$ is as defined above.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is $R_6OCH_2$- or $R_6OCH_2(R_6O)CH$-, wherein $R_6$ is as defined above.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: n is 0, 1 or 2.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is hydrogen.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is $C_1$-$C_6$ alkyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is $C_1$-$C_4$ alkyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is allyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is phenyl.

In some embodiments, the present application relates to the compound represented by the general Formula I, a stereoisomer, a pharmaceutically acceptable salt, a pharmaceutically acceptable ester, a pharmaceutically acceptable

hydrate or a pharmaceutically acceptable solvate thereof, wherein: $R_1$ is benzyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is methyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is ethyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is n-propyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is isopropyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is n-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is isobutyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is sec-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_1$ is tert-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is azido.

In some embodiments, the present application relates to the compound represented by general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is amino.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is amino substituted by one or two $R^a$ groups, wherein each $R^a$ is independently $C_1$-$C_6$ alkyl, allyl, phenyl, benzyl, formyl, acetyl benzoyl, trifluoroacetyl, methoxyformyl, tert-butoxyformyl or benzyloxyacyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is 5-membered or 6-membered nitrogen-containing heterocyclyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is 5-membered or 6-membered nitrogen-containing heterocyclyl substituted by $R^b$, wherein $R^b$ is $C_1$-$C_6$ alkyl, halogen or $-(CH_2)_mOH$, wherein m is 0, 1, 2, 3 or 4.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is 5-membered nitrogen-containing heterocyclyl substituted by $R^b$, wherein $R^b$ is $-(CH_2)_3OH$, and the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is 5-membered nitrogen-containing heterocyclyl substituted by $R^b$, wherein $R^b$ is $-(CH_2)_2OH$, and the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is 5-membered nitrogen-containing heterocyclyl substituted by $R^b$, wherein $R^b$ is $-CH_2OH$, and the 5-membered nitrogen-containing heteroaryl is triazolyl, tetrazolyl,

pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ is

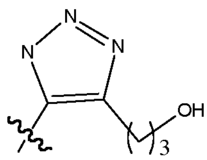

.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is azido, amino, amino substituted by one or two $R^a$, 5-membered or 6-membered nitrogen-containing heterocyclyl or 5-membered or 6-membered heterocyclyl nitrogen-containing heterocyclyl substituted by $R^b$, wherein each $R^a$ is independently $C_1$-$C_6$ alkyl, allyl, phenyl, benzyl, formyl, acetyl, benzoyl, trifluoroacetyl, methoxyformyl, tert-butyloxyformyl or benzyloxyacyl, $R^b$ is $C_1$-$C_6$ alkyl, halogen or $-(CH_2)_mOH$, wherein m is 0, 1, 2, 3 or 4.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_2$ and $R_3$ are each independently azido, amino or 5-membered nitrogen-containing heterocyclyl substituted by $R^b$, wherein $R^b$ is $-(CH_2)_mOH$, wherein m is 0, 1, 2 or 3, the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is 5-membered nitrogen-containing heterocyclyl substituted by $R^b$, wherein $R^b$ is $-(CH_2)_3OH$, and the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is 5-membered nitrogen-containing heterocyclyl substituted by $R^b$, wherein $R^b$ is $-(CH_2)_2OH$, and the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is 5-membered nitrogen-containing heterocyclyl substituted by $R^b$, wherein $R^b$ is $-CH_2OH$, and the 5-membered nitrogen-containing heteroaryl is triazolyl, tetrazolyl, pyrrolyl, tetrahydropyrrolyl, oxazolyl or imidazolyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is azido.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is amino.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is

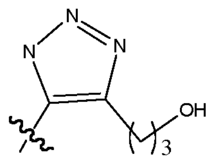

.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_3$ is

hydrate or a pharmaceutically acceptable solvate thereof, wherein: $R_4$ is hydroxyl, amino, guanidino, hydroxyl substituted by $R^c$, amino substituted by $R^c$, guanidino substituted by $R^c$, 5-membered or 6-membered nitrogen-containing heterocyclyl or 5-membered or 6-membered nitrogen-containing heterocyclyl substituted by $R^b$, wherein each $R^c$ is independently $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is hydroxyl, amino, guanidino, hydroxyl substituted by $R^c$, amino substituted by $R^c$, guanidino substituted by $R^c$, 5-membered or 6-membered nitrogen-containing heterocyclyl or 5-membered or 6-membered nitrogen-containing heterocyclyl substituted by $R^b$, wherein each $R^c$ is independently $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is hydroxyl, hydroxyl substituted by $R^c$, or amino substituted by $R^c$, wherein each $R^c$ is independently methyl, ethyl, n-propyl, isopropyl, n-butyl, isobutyl, sec-butyl, tert-butyl, trimethylsilyl, triethylsilyl, triisopropylsilyl, tert-butyldimethylsilyl, formyl, acetyl, benzoyl, or trifluoroacetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is hydroxyl, hydroxyl substituted by $R^c$, or amino substituted by $R^c$, wherein each $R^c$ is independently methyl, ethyl, n-propyl, isopropyl, n-butyl, isobutyl, sec-butyl, tert-butyl, trimethylsilyl, triethylsilyl, triisopropylsilyl, tert-butyldimethylsilyl, formyl, acetyl, benzoyl, or trifluoroacetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is hydroxyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is tert-butyldimethylsilyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is benzoyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is acetylamino.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ is acetyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is hydroxyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is trifluoroacetyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is acetylamino.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_5$ is acetyloxy.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form a 5-membered or 6-membered oxygen-containing or nitrogen-containing heterocyclic ring.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which

they connect form a 5-membered oxygen-containing or nitrogen-containing heterocyclic ring.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form a 5-membered oxygen-containing heterocyclic ring.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form 1,3-dioxolane, 1,3-dioxolan-2-one, oxazolidine or oxazolidinone.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form dioxane or dioxanone.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form 1,3-dioxolane or 1,3-dioxolan-2-one.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_4$ and $R_5$ together with the carbon atoms to which they connect form 1,3-dioxolan-2-one.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is hydrogen.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereofs, wherein: $R_6$ is $C_1$-$C_6$ alkyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is $C_1$-$C_4$ alkyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is methyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is ethyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is n-propyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is isopropyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is n-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is isobutyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is sec-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is tert-butyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is allyl.

In some embodiments, the present application relates to the compound represented by the general Formula

I, a stereoisomer, a pharmaceutically acceptable salt, a pharmaceutically acceptable ester, a pharmaceutically acceptable hydrate or a pharmaceutically acceptable solvate thereof, wherein: $R_6$ is phenyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is benzyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is trimethylsilyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is triethyl silyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is triisopropylsilyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is tert-butyldimethylylsilyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is tert-butyldiphenylsilyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is formyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is acetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is benzoyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_6$ is trifluoroacetyl.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is hydrogen.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is

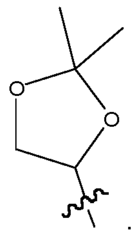

.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is $R_6OCH_2$-, wherein $R_6$ is as defined in the present application.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: $R_7$ is $R_6OCH_2(R_6O)CH$-, wherein the definition of $R_6$ is as defined in the present application.

In some embodiments, the present application relates to the compound represented by the general Formula I, a stereoisomer, a pharmaceutically acceptable salt, a pharmaceutically acceptable ester, a pharmaceutically acceptable

hydrate or a pharmaceutically acceptable solvate thereof, wherein: n is 0.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: n is 1.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: n is 2.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein: n is 3.

In some embodiments, the present application relates to the compound represented by the general Formula I, a stereoisomer, a pharmaceutically acceptable salt, a pharmaceutically acceptable ester, a pharmaceutically acceptable hydrate or a pharmaceutically acceptable solvate thereof, wherein the compound is selected from the group consisting of:

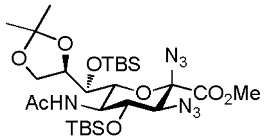 , 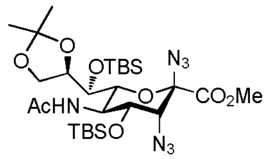 , 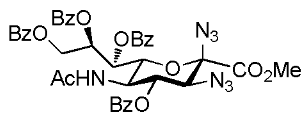 ,

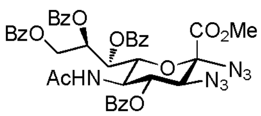 , 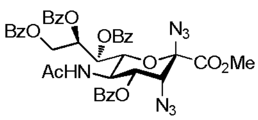 , 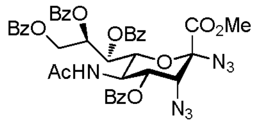 , 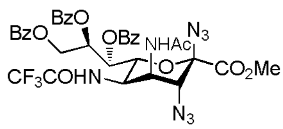 ,

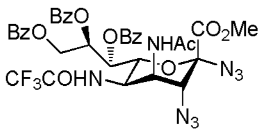 , 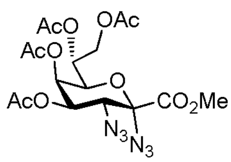 , 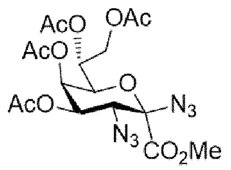 , 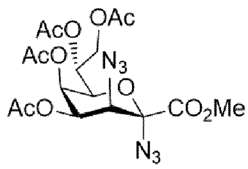 ,

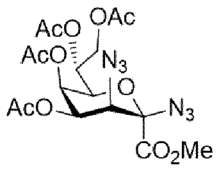 , 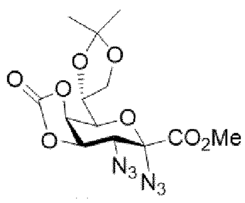 , 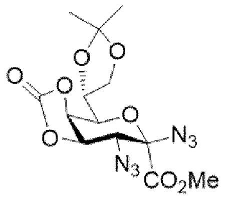 , 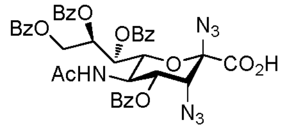 ,

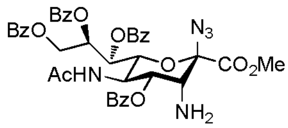 , 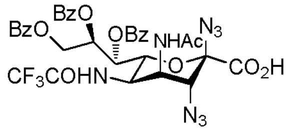 , 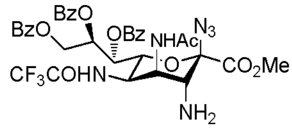 ,

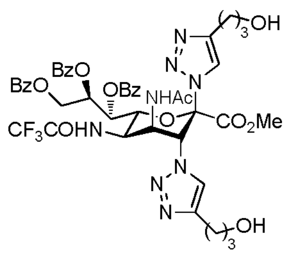 , 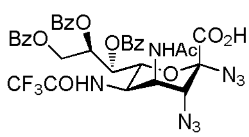 ,

and

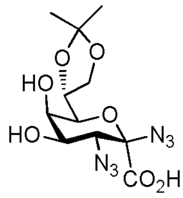

The present application also relates to a method for preparing the compound represented by the general Formula I, a stereoisomer, a pharmaceutically acceptable salt, a pharmaceutically acceptable ester, a pharmaceutically acceptable hydrate or a pharmaceutically acceptable solvent thereof, comprising:

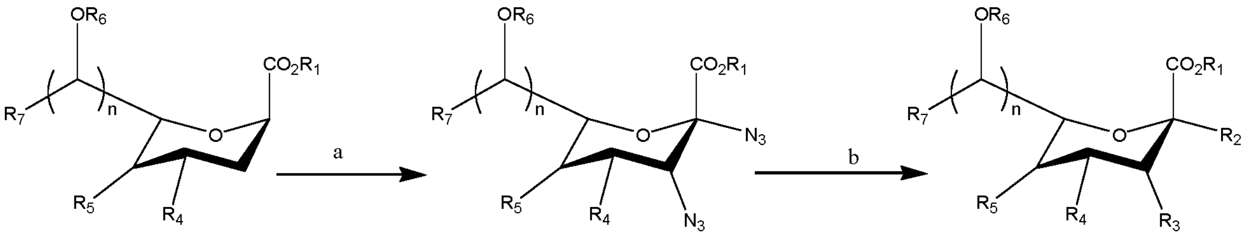


II III I

a) in a solvent, a compound represented by Formula II, a hypervalent iodine reagent and azidotrimethylsilane react under light conditions to generate a bis-azide represented by Formula III;

b) the bis-azide represented by Formula III generates the compound represented by the general Formula I through hydrolysis of the ester group, reduction of the azido group, removal of each protecting group on the hydroxyl or amino group, or formation of an azacycle,

wherein: the definition of $R_1$, $R_2$, $R_3$, $R_4$, $R_5$, $R_6$, $R_7$ is as described in any one of claims 1 to 5.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the hypervalent iodine reagent in step a) is selected from the group consisting of:

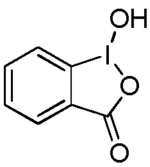 , 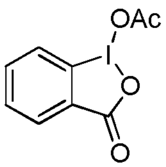 , 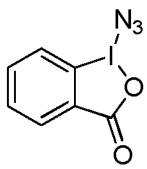 , 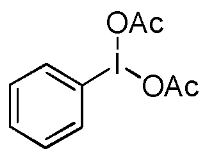 , 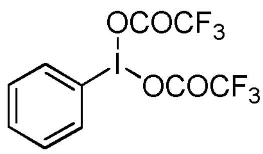 ,

and

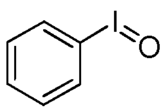 ,

etc., preferably is

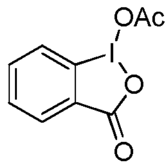
.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the solvent in step a) is selected from the group consisting of dichloromethane, acetone, dimethylsulfoxide, acetonitrile, etc., preferably is acetonitrile.

In some embodiments, the present application relates to a method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the light source for the light conditions in step a) is natural light or LED light of various colors, etc., preferably blue LED light.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the reaction in step a) is carried out at a temperature of 0-60°C, preferably under the light conditions and at a temperature of 20-40°C.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the hydrolysis of ester group in step b) is carried out in the presence of a base and a solvent, preferably, the base is selected from the group consisting of lithium hydroxide, sodium hydroxide, potassium hydroxide, sodium methoxide, sodium carbonate, potassium carbonate, sodium tert-butoxide, and potassium tert-butoxide, and the solvent is water or alcohol solvents, such as one or two selected from the group consisting of methanol, ethanol, isopropanol, and tert-butanol.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the reduction of azido group described in step b) is carried out in the presence of a reducing agent and a solvent, preferably, the reducing agent is selected from the group consisting of triphenylphosphine, trimethylphosphine, tributylphosphine, palladium carbon/hydrogen, palladium hydroxide/hydrogen, and Raney nickel/hydrogen, and the solvent is selected from the group consisting of water, tetrahydrofuran and alcohol solvents, such as one or two selected from the group consisting of methanol, ethanol, isopropanol, and tert-butanol.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the protecting group in step b) is removed in the presence of an acid or a base and a solvent, preferably, the acid is selected from the group consisting of hydrochloric acid, sulfuric acid, p-toluenesulfonic acid, and trifluoroacetic acid, the base is selected from the group consisting of sodium hydroxide, potassium hydroxide, sodium methoxide, sodium carbonate, potassium carbonate, sodium tert-butoxide, and potassium tert-butoxide, and the solvent is selected from water, dichloromethane, tetrahydrofuran and alcohol solvents, such as one or two selected from the group consisting of methanol, ethanol, isopropanol, and tert-butanol.

In some embodiments, the present application relates to the method for preparing the compound represented by general Formula I, a stereoisomer, a pharmaceutically acceptable salt, a pharmaceutically acceptable ester, a pharmaceutically acceptable hydrate or a pharmaceutically acceptable solvate thereof, wherein the formation of azacycle in step b) is carried out in the presence of an alkyne, a copper catalyst and a solvent, preferably, the alkyne is selected from terminal alkynes or internal alkynes of various lengths with or without various functional groups, the copper catalyst is selected from the group consisting of cuprous chloride, cuprous bromide, cuprous iodide, and copper sulfate/sodium ascorbate, and the solvent is selected from the group consisting of water, dichloromethane, tetrahydrofuran and alcoholic solvents, such as one or two selected from the group consisting of methanol, ethanol, isopropanol, and tert-butanol.

The present application also relates to a pharmaceutical composition, which comprises at least one of the compounds represented by general Formula I, a stereoisomer, a pharmaceutically acceptable salt, a pharmaceutically acceptable ester, a pharmaceutically acceptable hydrate or a pharmaceutically acceptable solvate thereof, and one or more pharmaceutically acceptable carriers or excipients.

The present application also relates to use of the compound represented by the general Formula I, a stereoisomer, a pharmaceutically acceptable salt, a pharmaceutically acceptable ester, a pharmaceutically acceptable hydrate

or a pharmaceutically acceptable solvate thereof or the pharmaceutical composition in the manufacture of a medicament for anti-virus (e.g., for anti-Zika virus, anti-rhinovirus).

The present application also relates to use of the compound represented by general Formula I, a stereoisomer, a pharmaceutically acceptable salt, a pharmaceutically acceptable ester, a pharmaceutically acceptable hydrate or a pharmaceutically acceptable solvent thereof or the pharmaceutical composition in the manufacture of a medicament for inhibiting the replication or reproduction of a virus (e.g., Zika virus, rhinovirus) in a cell (e.g., a cell of mammal).

The present application also relates to use of the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition in the manufacture of a medicament as an inhibitor against a virus (e.g., Zika virus, rhinovirus).

The present application also relates to use of the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition in the manufacture of a medicament for the prevention and/or treatment of a disease or infection caused by a virus (e.g., Zika virus, rhinovirus).

The present application also relates to the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition, for use as a medicament for anti-virus (e.g., anti-Zika virus, anti-rhinovirus).

The present application also relates to the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition, for use in inhibiting the replication or reproduction of a virus (e.g., Zika virus, rhinovirus) in a cell (e.g., a cell of mammal).

The present application also relates to the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition, for use as an inhibitor against a virus (e.g., Zika virus, rhinovirus).

The present application also relates to the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition, for use in the prevention and/or treatment of a disease or infection caused by a virus (e.g., Zika virus, rhinovirus).

The present application also relates to a method for preventing and/or treating a disease or infection caused by a virus (e.g., Zika virus, rhinovirus) in a mammal in need, comprising administering to the mammal in need a therapeutically and/or prophylactically effective amount of the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition.

The present application also relates to a method for inhibiting the replication or reproduction of a virus (e.g., Zika virus, rhinovirus) in a mammal in need, comprising administering to the mammal in need a therapeutically and/or prophylactically effective amount of the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition.

The present application also relates to a method for inhibiting the replication or reproduction of a virus (e.g., Zika virus, rhinovirus) in a cell (e.g., a cell of mammal), comprising contacting the cell with the compound represented by the general Formula I, a stereoisomer, a pharmaceutically acceptable salt, a pharmaceutically acceptable ester, a pharmaceutically acceptable hydrate or a pharmaceutically acceptable solvate thereof or the pharmaceutical composition.

In some embodiments, the mammal described in the present application comprises bovine, equine, caprinae, suidae, canidae, feline, rodent, primate, among which the preferred mammal is a human, a cat, a dog or a pig.

Definition of Substituent

The term "$C_{1-6}$ alkyl" as used herein refers to a saturated linear or branched monovalent hydrocarbyl having 1 to 6 carbon atoms. Typical examples of "$C_{1-6}$ alkyl" include, but are not limited to, methyl, ethyl, n-propyl, isopropyl, n-butyl, isobutyl, tert-butyl, n-pentyl, tert-pentyl, neopentyl, hexyl, etc.

The term "hydroxy" as used herein means -OH.

The term "halogen" as used herein refers to fluorine, chlorine, bromine or iodine. Preferred halogen group is fluorine, chlorine or bromine.

The term "5-membered or 6-membered nitrogen-containing heterocyclyl" as used herein refers to an aliphatic or aromatic heterocyclyl having 5 or 6 ring members and containing at least one nitrogen atom. Typical examples of the

"5-membered or 6-membered nitrogen-containing heterocyclyl" include, but are not limited to, triazole, tetrazole, pyrrole, tetrahydropyrrole, piperidine, pyridine, oxazole, imidazole, and the like.

The term "5-membered or 6-membered oxygen-containing or nitrogen-containing heterocyclyl" as used herein refers to an alicyclic or aromatic heterocyclyl having 5 or 6 ring members and containing at least one oxygen atom or one nitrogen atom. Typical examples of "5- or 6-membered oxygen-containing heterocyclyl" include, but are not limited to, 1,3-dioxolane, 1,3-dioxolan-2-one, dioxane, dioxanone, oxazolidine or oxazolidinone, etc.

In some embodiments, the pharmaceutically acceptable salt of the compound represented by the general Formula I described in the present application comprises its inorganic or organic acid salts, and inorganic or organic base salts, and the present application relates to all forms of the above salts. The pharmaceutically acceptable salt of the compound represented by the general Formula I includes but not limited to: sodium salt, potassium salt, calcium salt, lithium salt, meglumine salt, hydrochloride, hydrobromide, hydroiodide, nitrate, sulfate, hydrogensulfate, phosphate, hydrogenphosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, esilate, benzenesulfonate, p-toluenesulfonate and pamoate, etc.

In some embodiments, when the compound represented by the general Formula I described in the present application comprises hydroxyl, it can form a pharmaceutically acceptable ester with an organic acid or an inorganic acid, and the pharmaceutically acceptable ester includes esters that can be hydrolyzed in vivo, such as phosphate ester, sulfate ester, nitrate ester, formate ester, acetate ester, propionate ester, butyrate ester, valerate ester, and hexanoate ester.

In some embodiments, when the compound represented by the general Formula I described in the present application comprises carboxyl, it can also form a pharmaceutically acceptable ester with an alcohol compound, and the pharmaceutically acceptable ester includes esters that can be hydrolyzed in vivo, such as methyl ester, ethyl ester, propyl ester, n-butyl ester, and tert-butyl ester.

The carriers described in the present application include, but are not limited to: ion exchanger, aluminum oxide, aluminum stearate, lecithin, serum protein such as human albumin, buffer substance such as phosphate, glycerol, sorbic acid, potassium sorbate, mixture of partial glycerides of saturated vegetable fatty acids, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethyleneglycol, sodium carboxymethylcellulose, polyacrylate, beeswax, lanolin.

The term "excipient" used in the present application refers to an additive other than the main drug in a pharmaceutical preparation, which is stable in nature, has no incompatibility with the main drug, does not produce side effect, does not affect the curative effect, is not easily deformed, cracked, mildewed, and damaged by worms at room temperature, is harmless to the human body, has no physiological effects, does not produce chemical or physical effects with the main drug, and does not affect the content determination of the main drug, etc. For example, the binder, filler, disintegrant, lubricant in tablet; and preservative, antioxidant, flavoring agent, fragrance, cosolvent, emulsifier, solubilizer, osmotic pressure regulator, colorant, etc. in oral liquid preparation can be called excipients.

The pharmaceutical composition described in the present application can be administered through various routes, such as oral tablet, capsule, powder, oral liquid, injection and transdermal preparation. The above-mentioned medicament in various preparation forms can be prepared according to conventional methods in the pharmaceutical field. According to conventional pharmaceutical practices, the pharmaceutically acceptable carriers include diluent, filler, disintegrant, wetting agent, lubricant, coloring agent, flavoring agent or other conventional additive. Typical pharmaceutically acceptable carriers include, for example, microcrystalline cellulose, starch, crospovidone, povidone, polyvinylpyrrolidone, maltitol, citric acid, sodium lauryl sulfonate or magnesium stearate, etc.

According to the present application, the pharmaceutical composition can be administered in any of the following routes: oral administration, spray inhalation, rectal administration, nasal cavity administration, buccal administration, vaginal administration, topical administration, parenteral administration such as subcutaneous, intravenous, intramuscular, intraperitoneal, intrathecal, intraventricular, intrasternal and intracranial injection or infusion, or administration with the aid of an explanted reservoir.

As used herein, the "effective amount" refers to an amount that is sufficient to prevent or treat a disease in a patient but low enough to avoid serious side effects (at a reasonable benefit/risk ratio) within the scope of sound medical judgment. The prophylactically or therapeutically effective amount of compound will depend on the specific compound selected (e.g., considering the potency, effectiveness, and half-life of the compound), the route of administration selected, the disease to be prevented or treated, the severity of the disease to be prevented or treated, the age, size, weight and physical sickness of the patient to be treated, the medical history of the patient to be treated, the duration of prophylaxis or treatment, the nature of concurrent therapy, the desired effect of prophylaxis or treatment, etc., but still be routinely determined by those skilled in the art.

In addition, it should be pointed out that the specific dosage and usage of the compound of general Formula (I) described in the present application for different patients depend on many factors, including the patient’s age, body

weight, gender, natural health status, nutritional status, activity intensity of the compound, time of administration, metabolic rate, severity of symptoms, and subjective judgment of treating physicians. A dose of 0.001-1000 mg/kg body weight/day is preferably used here.

## Specific Models for Carrying Out the Invention

The present application can be further described by the following examples and test examples. However, the scope of the present application is not limited to the following examples or test examples. Those skilled in the art can understand that various changes and modifications can be made to the present application without departing from the spirit and scope of the present application. The present application provides a general and/or specific description of the materials and test methods used in the tests. Although many materials and operating methods used to achieve the purpose of the present application are well known in the art, the present application is still described herein as much detail as possible.

For all the following examples, standard operation and purification methods known to those skilled in the art can be used. All temperatures are expressed in °C (Celsius) unless otherwise stated.

In order to further describe the technical solution of the present application, some examples are given below, but the protection scope of the present application is not limited to the following description.

Example 1: Synthesis of Compound **4a** and **4b**

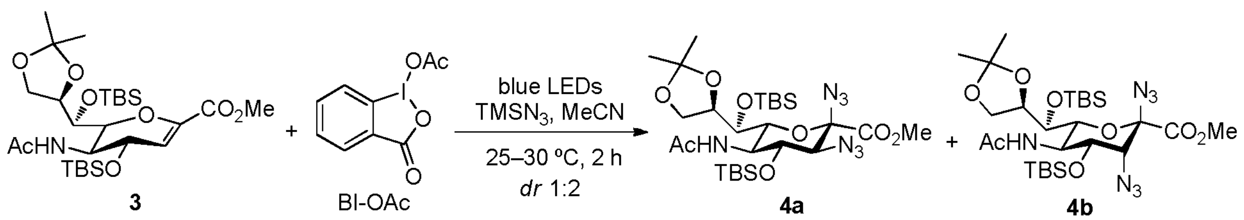


3 + BI-OAc → 4a + 4b

The unsaturated glycal compound **3** (201 mg, 0.351 mmol, 1.0 eq.) and BI-OAc (215 mg, 0.702 mmol, 2.0 eq.) were placed in a dry round-bottomed flask, after replacement with argon by suction, onsite-dried MeCN (3.5 mL) was added for dissolution, and $TMSN_3$ (138 μL, 1.05 mmol, 3.0 eq.) was added. The reaction was carried out under stirring and the illumination of 34W blue LED light, and the temperature was controlled at 25-30°C. After 2 h of reaction, TLC detection showed that the raw materials disappeared completely. A saturated KHCOs solution was added to the reaction solution to quench the reaction, then the reaction solution was diluted with EtOAc, and stirred vigorously at room temperature for 5 min, then extracted with EtOAc four times, the organic phases were combined and washed with water and saturated saline solution, respectively, dried with anhydrous $Na_2SO_4$, filtered, concentrated under reduced pressure, the crude product was separated and purified by silica gel column chromatography (petroleum ether : EtOAc = 12 : 1 to 8 : 1 v/v) to obtain white solid **4a** (65.3 mg, 28%) and **4b** (131 mg, 57%).

Compound **4a:** $^1$H NMR (400 MHz, $CDCl_3$) δ: 5.26 (d, *J* = 8.4 Hz, 1H), 4.41 (dd, *J* = 10.8, 1.2 Hz, 1H), 4.14 (q, *J* = 6.4 Hz, 1H), 4.07 - 3.86 (m, 3H), 3.92 (s, 3H), 3.85 -3.75 (m, 1H), 3.70 - 3.55 (m, 2H), 1.98 (s, 3H), 1.39 (s, 3H), 1.29 (s, 3H), 0.89 (s, 18H), 0.17 (s, 3H), 0.13 (s, 6H), 0.06 (s, 3H); $^{13}$C NMR (150 MHz, $CDCl_3$) δ: 169.6, 166.3, 108.6, 91.4, 76.0, 73.2, 71.5, 70.6, 66.4, 66.3, 53.9, 53.6, 26.6, 25.9, 25.6, 24.8, 23.9, 18.5, 18.0, -3.6, -4.0, -4.6, -4.6; [α]25 D = -16.0 (c 0.4, $CHCl_3$); IR (neat): $v_{max}$ = 2955, 2930, 2858, 2112, 1757, 1666, 1253, 1120, 1069, 1023, 836, 777, 736; HRMS: calcd. for $C_{27}H_{51}N_7NaO_8Si_2$[M + Na]$^+$ *m/z* 680.3235; found *m/z* 680.3249.

Compound **4b**: $^1$H NMR (400 MHz, $CDCl_3$) δ: 5.29 (d, *J* = 8.0 Hz, 1H), 4.73 (dd, *J* = 10.0, 3.6 Hz, 1H), 4.59 (d, *J* = 10.8 Hz, 1H), 4,24 - 4.16 (m, 1H), 4.07 - 4.01 (m, 2H), 3.97 - 3.83 (m, 2H), 3.85 (s, 3H), 3.67 - 3.55(m, 1H), 1.96 (s, 3H), 1.40 (s, 3H), 1.29 (s, 3H), 0.92 (s, 9H), 0.91 (s, 9H), 0.15 (s, 3H), 0.13 (s, 3H), 0.12 (s, 3H), 0.07 (s, 3H); $^{13}$C NMR (150 MHz, $CDCl_3$) δ: 170.2, 165.4, 108.1, 91.0, 72.7, 70.6, 67.8, 65.7, 64.9, 53.2, 50.8, 29.7, 26.4, 25.9, 25.6, 24.7, 23.9, 18.5, 17.9, - 3.4, -4.1, -4.4, -5.1; [α]25 D = -0.9 (c 0.4, $CHCl_3$); IR (neat): $v_{max}$ = 2955, 2930, 2858, 2111, 1758, 1668, 1256, 1068, 1039, 837, 779, 734; HRMS calcd. for $C_{27}H_{51}N_7NaO_8Si_2$[M + Na]$^+$ *m/z* 680.3235; found *m/z* 680. 3235.

Example 2: Synthesis of Compound **6a-d**

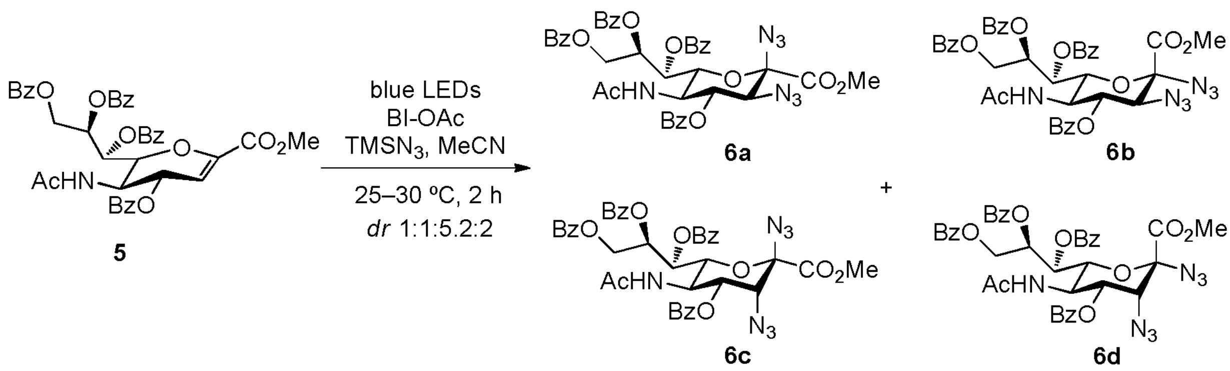


5 6a 6b + 6c 6d

The preparation method was the same as in Example 1. Compound **5** (2.03 g, 2.82 mmol, 1.0 eq.), BIOAc (1.72 g, 5.63 mmol, 2.0 eq.), $TMSN_3$ (1.11 mL, 8.45 mmol, 3.0 eq.) were reacted in dry MeCN. The crude product was separated and purified by silica gel column (petroleum ether: acetone = 4:1 v/v) to obtain white solid mixture **6** (1.92 g, 85%), which was separated by silica gel column (petroleum ether : EtOAc = 4 : 1 to 2 : 1 v/v) to obtain 1.50 g of a mixture of Compounds **6a, 6b** and **6c** and pure Compound **6d** (416 mg), and the mixture of the three was separated by analytical HPLC (Waters e2695, 2489 UV Detector, ZORBAX 300SB-C8, flow rate: 1 mL/min, temperature: 25°C, mobile phase was water : methanol = 60:40→40:60→30:70→20:80) to obtain three isomers in a ratio of **6a : 6b : 6c** = 1 : 1 : 5.2, in combination with the mass ratio analysis obtained by silica gel column purification, it could be known that the ratios of the four isomers were: **6a : 6b : 6c : 6d** = 1 :1 : 5. 2 : 2. The mixture of Compounds **6a, 6b** and **6c** could be separated by repeated silica gel column chromatography (petroleum ether: acetone = 8 : 1 v/v and petroleum ether: EtOAc = 5 : 1 v/v) and preparative silica gel plate separation (petroleum ether: EtOAc = 1.5 : 1 v/v) to obtain pure Compounds **6a** (52.3 mg), **6b** (32.8 mg) and **6c** (638 mg), and partial mixture, respectively.

Compound **6a:** $^1H$ NMR (400 MHz, $CDCl_3$) *δ*: 8.09 - 7.91 (m, 8H), 7.63 - 7.32 (m, 12H), 5.90 (dd, *J* = 6.4, 1.6 Hz, 1H), 5.87 - 5.81 (m, 1H), 5.64 (d, *J* = 9.6 Hz, 1H), 5.55 (t, *J* =2.0 Hz, 1H), 4.94 (dd, *J* = 12.4, 3.2 Hz, 1H), 4.64 (dd, *J* = 10.8, 2.0 Hz, 1H), 4.50 - 4.35 (m, 2H), 4.21 (d, *J* = 10.0 Hz, 1H), 3.96 (s, 3H), 1.78 (s, 3H); $^{13}C$ NMR (100 MHz, $CDCl_3$) δ: 170.0, 166.6, 166.0, 165.6, 165.2, 165.2, 133.8, 133.5, 133.4, 133.1, 130.0, 129.9, 129.9, 129.7, 129.5, 129.4, 129.2, 128.6, 128.6, 128.5, 128.3, 91.6, 72.9, 71.5, 70.6, 68.4, 62.9, 62.8, 54.1, 49.8, 23.0; [α]25 D = +6.1 (*c* 0.8, $CHCl_3$); IR (neat): $v_{max}$ = 2967, 2132, 2113, 1722, 1451, 1257, 1091, 1067, 1024, 800, 707, 686; HRMS calcd. for $C_{40}H_{35}N_7NaO_{12}$ $[M + Na]^+$ *m*/*z* 828.2241; found *m*/*z* 828.2232.

Compound **6b:** $^1H$ NMR (400 MHz, $CDCl_3$) δ: 8.17 - 7.88 (m, 8H), 7.65 - 7.31 (m, 12H), 5.96 - 5.76 (m, 3H), 5.53 (d, *J* = 9.6 Hz, 1H), 4.79 (dd, *J* = 12.4, 2.8 Hz, 1H), 4.67 (dd, *J* = 10.8, 2.0 Hz, 1H), 4.49 - 4.32 (m, 2H), 3.75 (d, *J* = 10.0 Hz, 1H), 3.48 (s, 3H), 1.80 (s, 3H); $^{13}C$ NMR (100 MHz, $CDCl_3$) δ: 170.0, 166.5, 166.0, 165.6, 165.3, 164.8, 133.7, 133.5, 133.0, 130.0, 129.9, 129.9, 129.7, 129.6, 129.2, 129.2, 128.6, 128.6, 128.5, 128.3, 91.2, 73.8, 71.5, 69.3, 67.9, 65.7, 63.0, 53.3, 49.3, 23.0; [α]25 D = +29.5 (c 0.9, $CHCl_3$); IR (neat): $v_{max}$ = 3364, 2963, 2932, 2114, 1726, 1249, 1093, 1068, 1026, 709; HRMS calcd. for $C_{40}H_{35}N_7NaO_{12}$ $[M + Na]^+$ *m*/*z* 828.2241; found 828.2235.

Compound **6c**: $^1H$ NMR (400 MHz, $CDCl_3$) δ: 8.18 - 8.13 (m, 2H), 8.05 - 7.98 (m, 4H), 7.98 - 7.92 (m, 2H), 7.64 - 7.45 (m, 6H), 7.45 - 7.31 (m, 6H), 5.94 - 5.86 (m, 2H), 5.84 (dd, *J* = 10.4, 3.2 Hz, 1H), 5.69 - 5.94 (m, 1H), 5.11 (dd, *J* = 12.4, 2.8 Hz, 1H), 4.68 (dd, *J* = 10.8, 2.0 Hz, 1H), 4.59 (dd, *J* = 12.4, 6.4 Hz, 1H), 4.49 (q, *J* = 10.4 Hz, 1H), 4.36 (d, *J* = 3.2 Hz, 1H), 3.96 (s, 3H), 1.81 (s, 3H); $^{13}C$ NMR (100 MHz, $CDCl_3$) δ: 170.1, 166.1, 166.0, 165.7, 165.6, 164.6, 133.8, 133.5, 133.3, 133.0, 130.1, 130.0, 129.9, 129.7, 129.7, 129.5, 129.2, 128.6, 128.6, 128.4, 128.4, 128.3, 91.2, 72.78, 71.5, 670.0, 69.0, 63.1, 62.5, 53.7, 45.9, 23.2; [α]25 D = -1.3 (c 0.7, $CHCl_3$); IR (neat): $v_{max}$ = 3375, 2970, 2924, 2112, 1723, 1695, 1257, 1092, 1068, 1026, 803, 709; HRMS calcd. for $C_{40}H_{35}N_7NaO_{12}$ $[M + Na]^+$ *m*/*z* 828.2241; found *m*/*z* 828.2232.

Compound **6d:** $^1H$ NMR (400 MHz, $CDCl_3$) δ: 8.19 - 8.13 (m, 2H), 8.07 - 7.98 (m, 4H), 7.97 - 7.92 (m, 2H), 7.67 - 7.32 (m, 12H), 5.97 - 5.90 (m, 1H), 5.86 (dd, *J* = 7.6, 1.6 Hz, 1H), 5.80 (dd, *J* = 10.8, 3.2 Hz, 1H), 5.64 - 5.55 (m, 1H), 4.95 (dd, *J* = 12.4, 3.2 Hz, 1H), 4.64 (dd, *J* = 10.8, 1.6 Hz, 1H), 4.59 - 4.49 (m, 2H), 4.20 - 4.08 (m, 1H), 3.62 (s, 3H), 1.85 (s, 3H); $^{13}C$ NMR (100 MHz, $CDCl_3$) δ: 170.4, 166.0, 165.9, 165.9, 165.7, 165.3, 133.8, 133.7, 133.3, 133.0, 130.2, 123.0, 129.9, 129.7, 129.6, 129.0, 128.7, 128.6, 128.5, 128.3, 89.5, 73.0, 69.9, 69.4, 68.7, 63.1, 62.8, 53.7, 46.6, 23.3; [α]25 D = +41.2 (*c* 0.7, $CHCl_3$); IR (neat): $v_{max}$ = 3375, 2963, 2111, 1724, 1255, 1106, 1094, 1069, 1026, 709; HRMS calcd. for $C_{40}H_{35}N_7NaO_{12}$ $[M + Na]^+$ *m*/*z* 828.2241; found *m*/*z* 828.2237.

Example 3: Synthesis of Compounds **8a** and **8b**

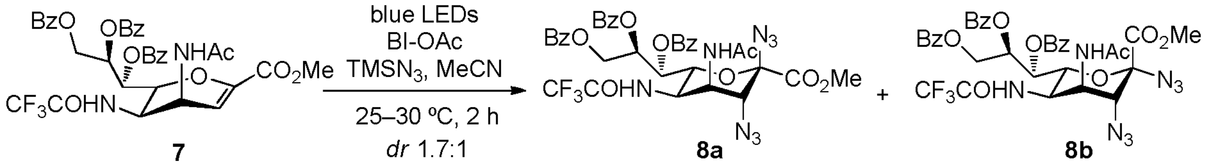


7 8a 8b

The preparation method was the same as in Example 1. Compound **7** (4.21 g, 5.91 mmol, 1.0 eq.), BIOAc (3.62 g, 11.8 mmol, 2.0 eq.) and $TMSN_3$ (2.33 mL, 17.7 mmol, 3.0 eq.) were reacted in dry MeCN. The crude product was separated and purified by silica gel column (petroleum ether: EtOAc = 3 : 1 to 2 : 1 v/v) to obtain white solids **8a** (1.27 g, 57%) and **8b** (0.732 g, 32%).

Compound **8a:** $^1$H NMR (400 MHz, $CDCl_3$) $\delta$: 8.10 - 7.98 (m, 4H), 7.97 - 7.90 (m, 2H), 7.65 - 7.31 (m, 9H), 7.16 - 7.07 (m, 1H), 6.72 (d, *J* = 8.8 Hz, 1H), 5.91 - 5.81 (m, 2H), 5.12 (dd, *J* = 12.8, 2.4 Hz, 1H), 4.93 (d, *J* = 10.8 Hz, 1H), 4.70 - 4.61 (m, 1H), 4.53 (dd, *J* = 12.4, 5.6 Hz, 1H), 4.35 - 4.25 (m, 1H), 4.02 (d, *J* = 3.6 Hz, 1H), 3.94 (s, 3H), 2.01 (s, 3H); $^{13}$C NMR (100 MHz, $CDCl_3$) $\delta$: 171.1, 166.1, 166.0, 165.3, 164.4, 158.4, 158.1, 157.7, 157.3, 133.8, 133.6, 133.1, 130.1, 129.9, 129.6, 129.5, 129.2, 128.8, 128.6, 128.6, 128.5, 128.3, 119.7, 116.9, 114.0, 111.1, 91.2, 71.4, 68.9, 68.5, 62.7, 61.1, 53.7, 49.8, 44.6, 23.2; [α]25 D = +63.1 (c 1.7, $CHCl_3$); IR (Neat): $v_{max}$ = 2114, 1725, 1259, 1088, 1068, 735, 709; HRMS calcd. for $C_{35}H_{31}F_3N_8NaO_{11}$ $[M + Na]^+$ *m/z* 819.1962; found *m/z* 819.1954.

Compound **8b:** $^1$H NMR (400 MHz, $CDCl_3$) $\delta$: 8.09 - 7.98 (m, 4H), 7.94 (d, *J* = 7.6 Hz, 2H), 7.64 - 7.49 (m, 4H), 7.49 - 7.41 (m, 3H), 7.41 - 7.32 (m, 2H), 6.71 - 6.56 (m, 1H), 5.92 - 5.85 (m, 1H), 5.80 (dd, *J* = 7.6, 1.6 Hz, 1H), 5.22 (d, *J* = 11.2, Hz, 1H), 4.92 (dd, *J* = 12.8, 2.8 Hz, 1H), 4.61 (d, *J* = 7.2 Hz, 1H), 4.46 (dd, *J* = 12.4, 4.8 Hz, 1H), 4.33 - 4.20 (m, 1H), 4.15 (q, *J* = 6.8 Hz, 1H), 3.68 (s, 3H), 1.96 (s, 3H); $^{13}$C NMR (100 MHz, $CDCl_3$) $\delta$: 172.4, 166.4, 166.0, 165.6, 165.5, 159.2, 158.8, 158.4, 158.05, 134.0, 133.6, 133.1, 130.1, 129.8, 129.6, 129.4, 129.2, 128.7, 128.6, 128.3, 128.3, 119.8, 117.0, 114.1, 111.2, 89.6, 70.1, 69.9, 68.5, 62.5, 59.0, 53.6, 50.6, 46.0, 22.9; [α]25 D = +53.4 (c 1.0, $CHCl_3$); IR (neat): $v_{max}$ = 2116, 1726, 1263, 1247, 1091, 1069, 734, 708; HRMS calcd. for $C_{35}H_{31}F_3N_8NaO_{11}$ $[M + Na]^+$ *m/z* 819.1962; found *m/z* 819.1961.

Example 4: Synthesis of Compound **10a-d**

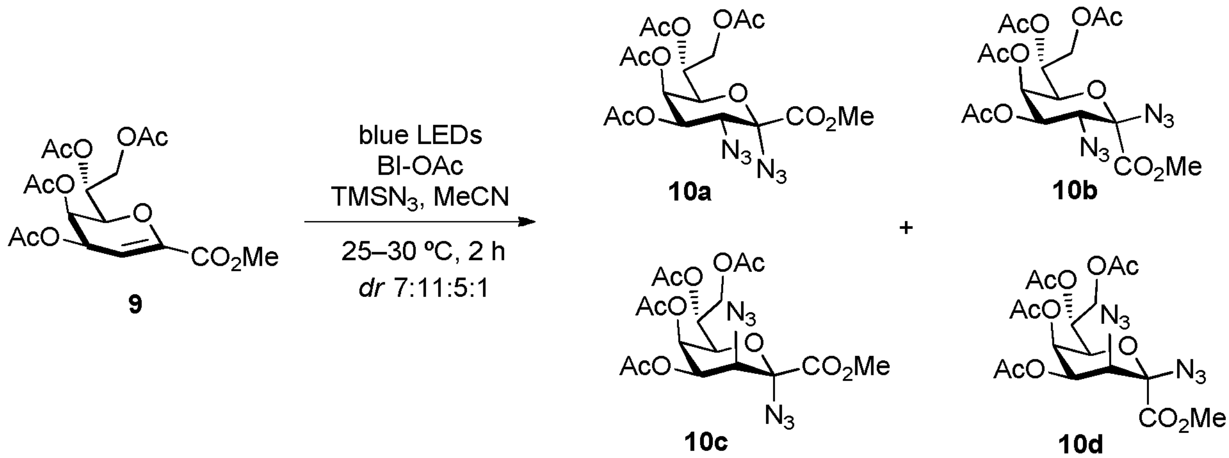


9 10a 10b 10c 10d

The preparation method was the same as in Example 1. Compound **9** (1.01 g, 2.51 mmol, 1.0 eq.), BIOAc (1.54 g, 5.02 mmol, 2.0 eq.) and $TMSN_3$ (0.991 mL, 7.53 mmol, 3.0 eq.) were reacted in dry MeCN. The crude product was purified by silica gel column (petroleum ether: acetone = 8: 1 v/v) to obtain 1.05 g of a mixed total product of four isomers (Compounds **10a, 10b, 10c, 10d**), which were separated by analytical HPLC (Waters e2695, 2424 ELS Detector, ZORBAX 300SB-C8, flow rate: 1 mL/min, temperature: 20°C, mobile phase was water : methanol = 60:40→50:50→40:60) to obtain four isomers in a ratio of **10a** : **10b** : **10c** : **10d** = 7 : 11 : 5 : 1. The total product was separated and purified by repeated silica gel column chromatography (petroleum ether : acetone = 13 : 1 v/v, petroleum ether: EtOAc = 7 : 1 v/v) to obtain white solid **10a** (34.0 mg), **10b** (163 mg), **10c** (194 mg), **10d** (41.3 mg), and partial mixture.

Compound **10a:** $^1$H NMR (600 MHz, $CDCl_3$) $\delta$: 5.48 (d, *J* = 1.8 Hz, 1H), 5.21 - 5.16 (m, 2H), 4.47 (dd, *J* = 12.6, 2.4 Hz, 1H), 4.43 (d, *J* = 9.6 Hz, 1H), 4.23 (d, *J* = 10.8 Hz, 1H), 4.09 (dd, *J* = 12.6, 3 Hz, 1H), 3.97 (s, 3H), 2.14 (s, 3H), 2.09 (s, 3H), 2.06 (s, 3H), 2.01 (s, 3H); $^{13}$C NMR (150 MHz, $CDCl_3$) $\delta$: 170.6, 170.1, 169.6, 169.5, 165.3, 91.6, 69.8,

69.2, 66.8, 65.4, 61.8, 58.0, 56.2, 54.1, 20.7, 20.6, 20.6; [α]25 D = +64.5 (c 0.11, $CHCl_3$); IR (neat): $v_{max}$ = 2927, 2118, 1752, 1437, 1370, 1226, 1076, 957, 750 $cm^{-1}$; HRMS calcd. for $C_{17}H_{22}N_6NaO_{11}$ $[M + Na]^+$ *m/z* 509.1244; found *m/z* 509.1253.

Compound 10b: $^1H$ NMR (400 MHz, $CDCl_3$) δ: 5.48 (d, *J* = 9.2 Hz, 2H), 5.14 (d, *J* = 9.2 Hz, 1H), 4.36 (t, *J* = 8.8 Hz, 2H), 4.22 (dd, *J* = 12, 2.8 Hz, 1H), 3.92 (s, 3H), 3.76 (d, *J* = 10 Hz, 1H), 2.14 (s, 3H), 2.07 (s, 3H), 2.04 (s, 3H), 2.01 (s, 3H); $^{13}C$ NMR (150 MHz, $CDCl_3$) *δ*: 170.3, 170.1, 169.5, 169.5, 165.7, 91.2, 71.5, 69.4, 67.4, 65.1, 61.8, 61.3, 53.6, 20.7, 20.6, 20.6, 20.6; [α]25 D = +32.5 c0.08, $CHCl_3$); IR (neat): $v_{max}$ = 2926, 2118, 1752, 1437, 1372, 1228, 1080, 804, 735 $cm^{-1}$; HRMS calcd. for $C_{17}H_{22}N_6NaO_{11}$ $[M + Na]^+$ *m/z* 509.1244; found *m/z* 509.1253.

Compound **10c:** $^1H$ NMR (400 MHz, $CDCl_3$) δ: 5.42 (d, *J* = 2.4 Hz, 2H), 5.34 (d, *J* = 10 Hz, 1H), 4.55 (dd, *J* = 12.4, 2 Hz, 1H), 4.42 (d, *J* = 9.6 Hz, 1H), 4.24 (dd, *J* = 12.8, 2.8 Hz, 1H), 4.07 (t, *J* = 2.0 Hz, 1H), 3.93 (s, 3H), 2.13 (s, 3H), 2.08 (s, 6H), 1.99 (s, 3H); $^{13}C$ NMR (150 MHz, $CDCl_3$) δ: 170.5, 170.5, 169.4, 169.2, 164.4, 91.5, 69.4, 67.3, 67.1, 63.3, 61.8, 58.5, 53.7, 20.7, 20.6, 20.6, 20.3; [α]25 D = +116.2 (c 0.32, $CHCl_3$); IR (neat): $v_{max}$ = 2959, 2118, 1748, 1438, 1372, 1223, 1053, 924, 732 $cm^{-1}$; HRMS calcd. for $C_{17}H_{22}N_6NaO_{11}$ $[M + Na]^+$ *m/z* 509.1244; found *m/z* 509.1231.

Compound **10d:** $^1H$ NMR (400 MHz, $CDCl_3$) δ: 5.34 (s, 1H), 5.30 (d, J = 9.6 Hz, 1H), 5.15 (t, *J* = 2.8 Hz, 1H), 4.46 (d, *J* = 12.4 Hz, 1H), 4.29 (dd, *J* = 12.4, 3.2 Hz, 1H), 4.22 (d, *J* = 3.2 Hz, 1H), 3.97 (d, *J* = 9.6 Hz, 1H), 3.92 (s, 3H), 2.16 (s, 3H), 2.11 (s, 3H), 2.09 (s, 3H), 1.98 (s, 3H); $^{13}C$ NMR (150 MHz, $CDCl_3$) δ: 170.5, 170.4, 169.3, 169.3, 165.8, 90.1, 71.9, 67.7, 67.3, 62.9, 61.7, 58.7, 53.9, 20.7, 20.6, 20.5, 20.4; [α]25 D = +101.8 (c 0.09, $CHCl_3$); IR (neat): $v_{max}$ = 2918, 2115, 1747, 1436, 1372, 1225, 1045, 923, 733 $cm^{-1}$; HRMS calcd. for $C_{17}H_{22}N_6NaO_{11}$ $[M + Na]^+$ *m/z* 509.1244; found *m/z* 509.1258.

Example 5: Synthesis of Compounds **12a** and **12b**

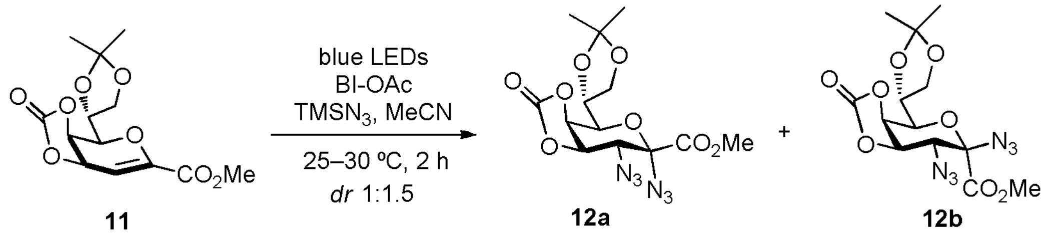


**11** **12a** **12b**

The preparation method was the same as in Example 1. Compound **11** (1.20 g, 4.00 mmol, 1.0 eq.), BIOAc (2.45 g, 8.00 mmol, 2.0 eq.) and $TMSN_3$ (1.58 mL, 12.0 mmol, 3.0 eq.) were reacted in dry MeCN. The crude product was separated and purified by silica gel column (petroleum ether: EtOAc = 8 : 1 to 5 : 1 v/v) to obtain white solids **12a** (537 mg, 35%) and **12b** (845 mg, 55%).

Compound **12a:** $^1H$ NMR (400 MHz, $CDCl_3$) δ: 4.91 (dd, *J* = 6.8, 2 Hz, 1H), 4.77 (t, *J* = 6.8 Hz, 1H), 4.37 - 4.32 (m, 1H), 4.21 (d, *J* = 6.8 Hz, 1H), 4.18 - 4.12 (m, 1H), 4.01 (d, *J* = 2 Hz, 1H), 3.99 - 3.95 (m, 1H), 3.94 (s, 3H), 1.44 (s, 3H), 1.36 (s, 3H); $^{13}C$ NMR (100 MHz, $CDCl_3$) *δ*: 165.1, 152.5, 110.2, 90.0, 74.5, 73.3, 72.7, 71.2, 67.0, 60.1, 54.2, 26.8, 24.8; [α]25 D = +76.7 (*c* 0.68, $CHCl_3$); IR (neat): $v_{max}$ = 2988, 2968, 2121, 1820, 1768, 1079, 846, 759 $cm^{-1}$; HRMS calcd. for $C_{13}H_{16}N_6NaO_8$ $[M + Na]^+$ *m/z* 407.0927; found *m/z* 407.0932.

Compound **12b:** $^1H$ NMR (400 MHz, $CDCl_3$) δ: 5.02 (dd, *J* = 8.0, 5.2 Hz, 1H), 4.96 (dd, *J* = 8.0, 1.6 Hz, 1H), 4.36 - 4.31 (m, 1H), 4.19 - 4.12 (m, 2H), 3.94 (s, 3H), 3.91 (d, *J* = 4.8 Hz, 1H), 3.79 (dd, *J* = 8.4, 1.6 Hz, 1H), 1.41 (s, 3H), 1.35 (s, 3H); $^{13}C$ NMR (100 MHz, $CDCl_3$) *δ*: 165.4, 152.7, 110.2, 89.3, 73.7, 72.8, 72.5, 72.4, 66.7, 61.2, 53.7, 26.9, 24.8; [α]25 D = +40.0 (c 0.08, $CHCl_3$); IR (neat): $v_{max}$ = 2988, 2967, 2111, 1820, 1767, 1078, 846, 759 $cm^{-1}$; HRMS calcd. for $C_{13}H_{16}N_6NaO_8$ $[M + Na]^+$ *m/z* 407.0927; found *m/z* 407.0915.

Example 6: Synthesis of Compound **13**

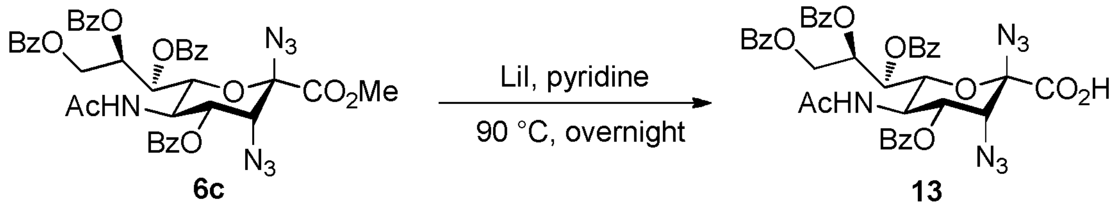


6c 13

Compound **6c** (50.3 mg, 0.062 mmol, 1.0 eq.) and LiI (83.1 mg, 0.621 mmol, 10.0 eq.) were placed in a dry test tube, after replacement with Ar gas by suction, 1 mL of pyridine was added for dissolution, stirred in an oil bath at 90°C overnight. The reaction solution was concentrated under reduced pressure, and purified by a silica gel column ($CH_2Cl_2$:MeOH = 20:1 v/v) to obtain a white solid product **13** (42.5 mg, 86%). $^1$H NMR (400 MHz, $CD_3OD$) *δ*: 8.17 (d, *J*= 7.2 Hz, 2H), 8.05 (d, *J* = 7.2 Hz, 2H), 8.00 (d, *J* = 7.2 Hz, 2H), 7.94 (d, *J* = 7.2 Hz, 2H), 7.69 - 7.34 (m, 12H), 6.05 - 5.93 (m, 2H), 5.54 (dd, *J* = 10.4, 3.6 Hz, 1H), 5.08 (dd, *J* = 12.4, 2.4 Hz, 1H), 4.68 - 4.47 (m, 4H), 4.44 (d, *J* = 3.6 Hz, 1H), 1.80 (s, 3H); $^{13}$C NMR (100 MHz, $CD_3OD$) δ: 173.3, 167.6, 167.1, 167.0, 166.8, 150.0, 134.7, 134.6, 134.6, 134.3, 131.3, 131.0, 131.0, 131.0, 130.9, 130.8, 130.7, 130.3, 129.7, 129.6, 129.6, 129.5, 73.3, 73.1, 71.6, 69.6, 64.9, 63.6, 46.0, 30.7, 22.7; [α]25 D = +21.4 (c 0.44, $CHCl_3$ : MeOH = 4:1); IR (neat): $v_{max}$ = 3398, 2119, 1725, 1662, 1261, 1090, 1069, 1025, 707, 686; HRMS calcd.for $C_{39}H_{33}N_7NaO_{12}$ [M + Na]$^+$ *m/z* 814.2085; found *m/z* 814.2055.

Example 7: Synthesis of Compound **14**

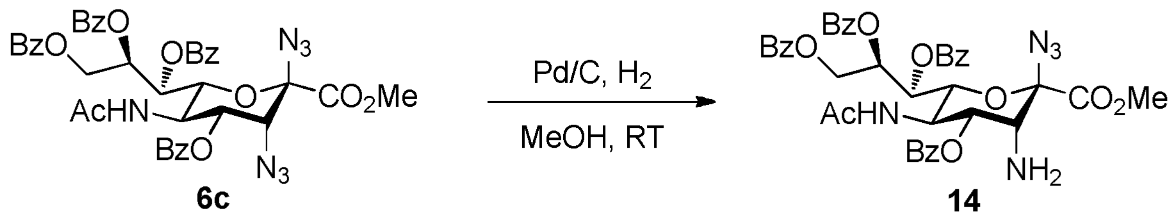


6c 14

Compound **6c** (140 mg, 0.174 mmol, 1.0 eq.) and Pd/C (14.0 mg) were placed in a round-bottomed flask, after replacement with $H_2$ by suction, 10 mL of methanol was added for dissolution, stirred at room temperature for 4 h. The reaction solution was filtered through diatomite, concentrated under reduced pressure, and purified by silica gel column (petroleum ether: EtOAc = 2:1 v/v) to obtain a white solid product **14** (110 mg, 81%). $^1$H NMR (400 MHz, $CDCl_3$) *δ*: 8.21 - 8.14 (m, 2H), 8.03 - 7.97 (m, 4H), 7.96 - 7.90 (m, 2H), 7.64 - 7.45 (m, 6H), 7.45 - 7.30 (m, 6H), 5.98 (dd, *J* = 10.8, 3.6 Hz, 1H), 5.95 - 5.80 (m, 1H), 5.84 (dd, *J* = 5.6, 2.0 Hz, 1H), 5.58 (d, *J* = 9.6 Hz, 1H), 5.19 (dd, *J* = 12.4, 2.8 Hz, 1H), 4.81 (dd, *J* = 10.4, 2.0 Hz, 1H), 4.56 (dd, *J* = 12.0, 7.6 Hz, 1H), 4.44 (q, *J* = 10.0 Hz, 1H), 4.32 (d, *J* = 3.6 Hz, 1H), 3.88 (s, 3H), 2.13 (s, 2H), 1.81 (s, 3H); $^{13}$C NMR (100 MHz, $CDCl_3$) δ: 170.3, 170.1, 166.4, 166.2, 165.9, 133.7, 133.4, 132.9, 130.2, 130.0, 129.8, 129.8, 129.6, 129.4, 129.4, 128.6, 128.5, 128.5, 128.5, 128.3, 86.3, 72.0, 71.2, 70.3, 69.5, 63.8, 63.7, 53.0, 46.5, 23.3; [α]25 D = +4.4 (c 0.50, $CHCl_3$); IR (neat): $v_{max}$ = 3378, 2928, 2855, 2111, 1718, 1261, 1107, 1094, 1069, 1026, 708; HRMS calcd. for $C_{40}H_{37}N_5NaO_{12}$ [M + Na]$^+$ *m/z* 802.2336; found *m/z* 802.2325.

Example 8: Synthesis of Compound **15**

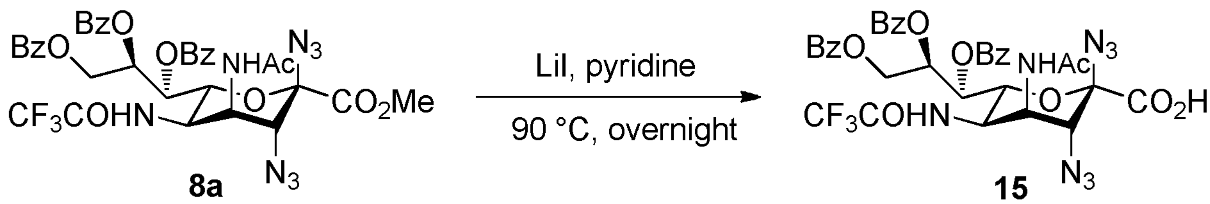


8a 15

The preparation method was the same as in Example 6. Compound **8a** (100 mg, 0.126 mmol, 1.0 eq.) and LiI (168.1 mg, 1.26 mmol, 10.0 eq.) were reacted in 2 mL of pyridine. The crude product was purified by silica gel column ($CH_2Cl_2$ : MeOH = 20 : 1 to 15 : 1 v/v) to obtain a white solid product **15** (74.7 mg, 76%). $^1$H NMR (400 MHz, $CD_3OD$)

δ: 8.13 (d, *J* = 7.6 Hz, 2H), 8.02 (d, *J* = 7.2 Hz, 2H), 7.93 (d, *J* = 7.2 Hz, 2H), 7.70 - 7.34 (m, 9H), 6.10 - 6.02 (m, 1H), 5.98 (dd, *J* = 8.0, 2.0 Hz, 1H), 5.16 (dd, *J* = 12.4, 2.0 Hz, 1H), 4.70 (dd, *J* = 11.2, 1.6 Hz, 1H), 4.65 - 4.55 (m, 3H), 4.49 (dd, *J* = 12.4, 4.8 Hz, 1H), 4.39 (dd, *J* = 11.2, 4.4 Hz, 1H), 4.00 (d, *J* = 3.2 Hz, 1H), 2.01 (s, 3H); $^{13}C$ NMR (150 MHz, $CD_3OD$) *δ*: 171.4, 168.3, 165.6, 164.9, 164.7, 157.1, 156.8, 132.8, 132.6, 132.4, 129.2, 129.0, 128.9, 128.7, 128.6, 128.5, 128.5, 127.7, 127.6, 127.5, 127.5, 127.5, 127.5, 127.4, 116.1, 114.2, 91.7, 69.5, 67.9, 66.9, 61.5, 61.3, 48.3, 42.9, 20.9; [α]25 D = +45.0 (*c* 0.50, $CHCl_3$); IR (neat): $v_{max}$ = 2117, 1718, 1654, 1452, 1259, 1177, 1091, 1068, 709; HRMS calcd. for $C_{34}H_{29}F_3N_8NaO_{11}$ $[M + Na]^+$ *m/z* 805.1806; found *m/z* 805.1803.

Example 9: Preparation of Compound **16:**

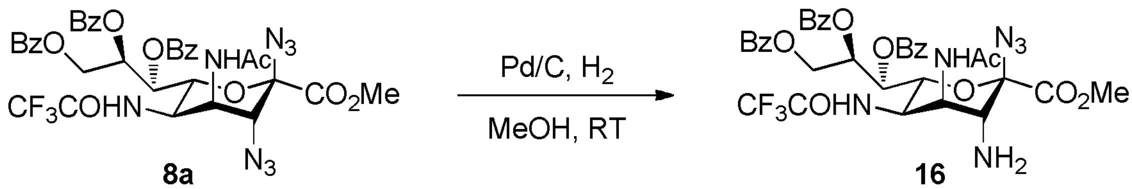


**8a** **16**

The preparation method was the same as in Example 7. Compound **8a** (100 mg, 0.126 mmol) and Pd/C (10.2 mg) were placed in a round-bottomed flask, after replacement with $H_2$ by suction, 5 mL of methanol was added for dissolution, stirred at room temperature for 3 h. The reaction solution was filtered through diatomite, concentrated under reduced pressure, and purified by silica gel column (petroleum ether: EtOAc = 1.5 : 1 v/v) to obtain a white solid product **16** (58.9 mg, 63%). $^1H$ NMR (400 MHz, $CDCl_3$) δ: 8.15 - 8.07 (m, 2H), 8.06 - 7.99 (m, 2H), 7.98 - 7.89 (m, 2H), 7.72 (d, *J* = 9.2 Hz, 1H), 7.64 - 7.32 (m, 9H), 6.76 (d, *J* = 9.2 Hz, 1H), 5.91 - 5.85 (m, 1H), 5.75 (dd, *J* = 4.8, 1.6 Hz, 1H), 5.31 (dd, *J* = 12.4, 2.4 Hz, 1H), 4.78 - 4.69 (m, 2H), 4.46 (dd, *J* = 12.4, 7.2 Hz, 1H), 4.43 - 4.35 (m, 1H), 3.89 (s, 3H), 3.84 (d, *J* = 3.2 Hz, 1H), 2.02 (s, 3H); $^{13}C$ NMR (100 MHz, $CDCl_3$) δ: 170.6, 170.0, 166.4, 166.4, 165.3, 158.0, 157.5, 157.1, 156.8, 133.6, 133.5, 133.1, 130.1, 129.9, 129.6, 129.6, 129.2, 129.0, 128.6, 128.6, 128.4, 116.9, 114.0, 86.0, 72.4, 69.2, 67.6, 63.8, 62.7, 53.2, 49.8, 44.5, 23.5; [α]25 D = +124.8 (*c* 0.40, $CHCl_3$); IR (neat): $v_{max}$ = 3377, 3287, 2115, 1718, 1259, 1250, 1089, 1068, 1025, 708; HRMS calcd. for $C_{35}H_{33}F_3N_6NaO_{11}$ $[M + Na]^+$ *m/z* 793.2057; found *m/z* 793.2049.

Example 10: Preparation of Compound **17:**

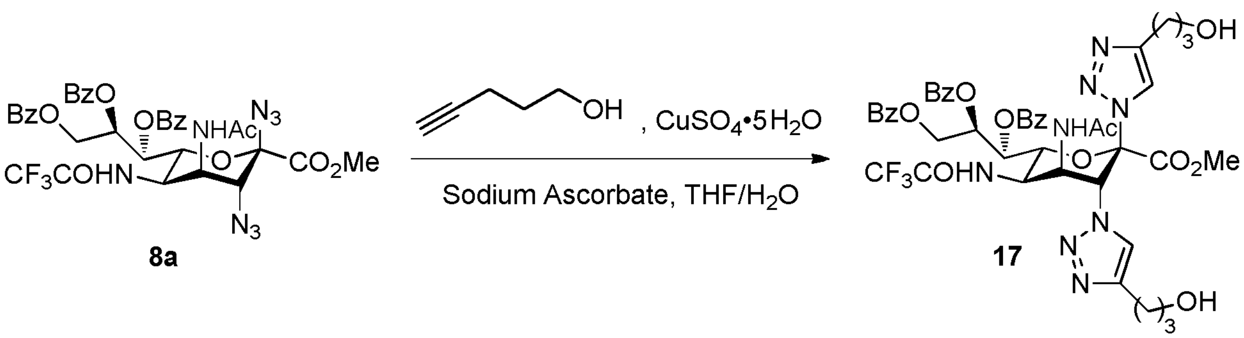


**8a** **17**

Compound **8a** (150 mg, 0.188 mmol, 1.0 eq.) was dissolved in a mixed solvent of THF and $H_2O$ (14 mL, 1 : 1 v/v), and then pentynyl alcohol (38.6 μL, 0.414 mmol, 2.2 eq.), $CuSO_4 \cdot 5H_2O$ (18.8 mg, 0.075 mmol, 0.4 eq.) and sodium ascorbate (149 mg, 0.754 mmol, 4.0 eq.) were added in sequence. The reaction was carried out under stirring at room temperature for 2 h, monitored by TLC until the completion of the reaction of the raw materials, and the reaction solution was directly concentrated under reduced pressure, first purified by a reverse-phase silica gel column (MeOH : $H_2O$ = 1 : 4 to 1 : 1 v/v), and then purified by normal phase silica gel column purification ($CH_2Cl_2$: MeOH = 15: 1 v/v) to obtain a white solid product **17** (151 mg, 83%). $^1H$ NMR (400 MHz, $CD_3OD$) δ: 8.25 - 8.18 (m, 2H), 8.02 (s, 1H), 7.93 - 7.87 (m, 4H), 7.85 (s, 1H), 7.74 - 7.53 (m, 5H), 7.46 (t, *J* = 8.0 Hz, 2H), 7.39 (t, *J* = 8.0 Hz, 2H), 6.95 (d, *J* = 8.0 Hz, 1H), 5.95 (dd, *J* = 8.0, 1.6 Hz, 1H), 5.78 - 5.71 (m, 1H), 5.17 - 5.06 (m, 2H), 5.02 (dd, *J* = 10.8, 6.8 Hz, 1H), 4.63 (dd, *J* = 10.8, 1.6 Hz, 1H), 4.60 (s, 1H), 4.39 (dd, *J* = 12.8, 4.8 Hz, 1H), 3.62 (s, 3H), 3.57 - 3.50 (m, 4H), 2.74 (t, *J* = 7.2 Hz, 2H), 2.49 (t,

*J* = 7.6 Hz, 2H), 1.88 - 1.70 (m, 4H), 1.82 (s, 3H); $^{13}C$ NMR (100 MHz, $CD_3OD$) δ: 173.7, 167.5, 166.7, 166.6, 165.4, 160.0, 159.6, 159.3, 158.9, 150.8, 148.3, 135.1, 134.9, 134.5, 131.3, 130.8, 130.7, 130.7, 130.4, 130.2, 129.9, 129.8, 129.7, 129.6, 129.5, 126.2, 123.0, 118.6, 115.7, 92.6, 71.3, 70.8, 69.8, 63.1, 62.0, 61.8, 58.5, 54.8, 49.9, 47.2, 33.1, 32.5, 22.8, 22.5, 22.4; [α]25 D = +36.4 (c 0.52, $CHCl_3$); IR (neat): $v_{max}$ = 3339, 2936, 1721, 1260, 1106, 1068, 708 $cm^{-1}$; HRMS calcd. for $C_{45}H_{47}F_3N_8NaO_{13}$ $[M + Na]^+$ *m/z* 987.3112; found *m/z* 987.3072.

Example 11: Preparation of Compound 18:

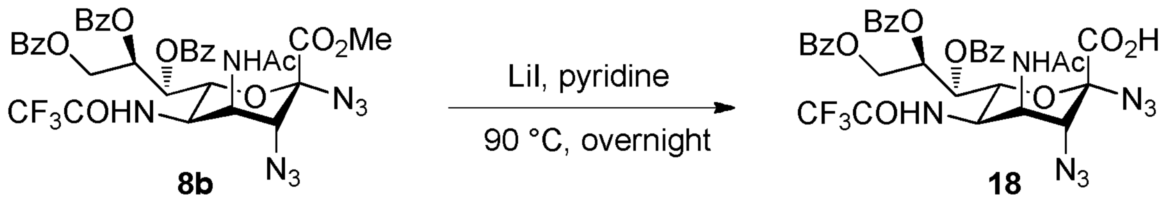


**8b** **18**

The preparation method was the same as in Example 6. Compound **8b** (52.3 mg, 0.066 mmol, 1.0 eq.) and LiI (87.9 mg, 0.657 mmol, 10.0 eq.) were reacted in 1 mL of pyridine. The crude product was purified by silica gel column ($CH_2Cl_2$ : MeOH = 20 : 1 to 10 : 1 v/v) to obtain a white solid product **18** (42.6 mg, 83%). $^1H$ NMR (400 MHz, $CD_3OD$) δ: 8.11 (dd, J = 8.0, 1.2 Hz, 2H), 7.96 (dd, J = 8.0, 1.2 Hz, 2H), 7.90 (dd, *J* = 8.4, 1.6 Hz, 2H); 7.68 - 7.30 (m, 9H), 5.98 - 5.85 (m, 2H), 5.10 (dd, *J* = 12.4, 3.2 Hz, 1H), 4.78 (dd, *J* = 10.4, 2.0 Hz, 1H), 4.70 (dd, *J* = 12.4, 6.4 Hz, 1H), 4.61 - 4.50 (m, 1H), 4.44 (t, *J* = 5.2 Hz, 1H), 4.21 (d, *J* = 4.8 Hz, 1H), 1.95 (s, 3H); $^{13}C$ NMR (100 MHz, $CD_3OD$) *δ*: 173.8, 172.6, 167.6, 167.0, 166.7, 159.5, 159.2, 158.8, 158.4, 134.7, 134.4, 134.3, 134.1, 131.1, 131.1, 131.0, 130.9, 130.8, 130.7, 130.6, 130.5, 130.5, 129.7, 129.6, 129.5, 129.5, 129. 4, 129.3, 121.5, 118.6, 115.8, 93.6, 72.6, 70.9, 63.8, 62.6, 50.2, 46.0, 22.9, 13.9; [α]25 D = +82.8 (c 0.40, $CHCl_3$); IR (neat): $v_{max}$ = 3726, 3083, 2889, 2114, 1722, 1647, 1262, 1177, 1093, 1069, 707; HRMS calcd. for $C_{34}H_{29}F_3N_8NaO_{11}$ $[M + Na]^+$ *m/z* 805.1806; found *m/z* 805.1811.

Example 12: Preparation of Compound **19:**

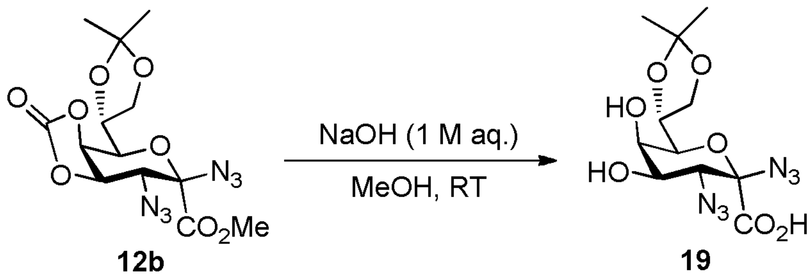


**12b** **19**

Compound **12b** (50.4 mg, 0.131 mmol, 1.0 eq.) was dissolved in MeOH (1 mL), 1M NaOH aqueous solution (0.131 mL) was added dropwise, after the dropping was completed, the reaction solution was stirred at room temperature for 1 h, and then $H^+$ ion exchange resin was added to regulate pH to neutral, the reaction solution was filtered, concentrated under reduced pressure, and purified by a reverse-phase silica gel column (MeOH : $H_2O$ = 1 : 9 v/v) to obtain a white solid product **19** (34.2 mg, 76%). $^1H$ NMR (400 MHz, $CD_3OD$) *δ*: 4.41 (dd, *J* = 10.4, 3.6 Hz, 1H), 4.34 - 4.25 (m, 1H), 4.08 (dd, *J* = 8.8, 6.0 Hz, 1H), 4.02 - 3.90 (m, 3H), 3.46 (d, *J*= 10.4 Hz, 1H), 1.35 (s, 3H), 1.32 (s, 3H); $^{13}C$ NMR (100 MHz, $CD_3OD$) δ: 171.7, 110.4, 94.4, 77.7, 74.6, 71.0, 69.3, 68.3, 66.3, 27.0, 25.6; [α]25 D = +91.2 (c 0.17, $CHCl_3$); IR (neat): $v_{max}$ = 3383, 2925, 2114, 1622, 1615, 1384, 1375, 1247, 1226, 1071, 1059, 842; HRMS calcd. for $C_{11}H_{16}N_6NaO_7$ $[M + Na]^+$ *m/z* 367.0978; found *m/z* 367.0961.

Example 13: Biological activity test

The nitrogen-containing derivatives of 3-deoxy-2-ketoaldonic acid obtained in the above examples were subjected to an antiviral test, and the results were shown in Table 1.

(1) Anti-Zika (ZIKV) virus activity test

Test object: Zika virus-infected BHK cells

Testing method:

① BHK cells (preserved by the Academy of Military Medical Sciences) were inoculated into a 96-well plate at a concentration of 5×10³ cells/well, and the cell culture medium was DMEM medium (purchased from Gibco Company, Cat. No. 11995065) containing 10% FBS (purchased from Gibco, Cat. No. 16000044), the plate was placed in a $CO_2$ incubator, and incubated at 37°C for 24 hours.

② The original medium in the 96-well plate was discarded, 100 μL of DMEM medium (purchased from Gibco, Cat. No. 11995065) containing 2% FBS (purchased from Gibco, Cat. No. 16000044) was taken and added into the cells. Then, the stock solution of the compound to be tested was serially diluted with the above-mentioned DMEM medium to 800 μM, 266.67 μM, 88.89 μM, 29.63 μM, 9.88 μM, 3.29 μM, 1.10 μM, 0.37 μM, and 50 μL was added to the cell culture plate. The stock solution of positive compound NITD008 (purchased from MCE Company, Cat. No. HY-12957) was serially diluted with the above DMEM medium to 80 μM, 26.67 μM, 8.89 μM, 2.96 μM, 0.99 μM, 0.33 μM, 0.11 μM, 0.04 μM, and 50 μL was added to the cell culture plate. Finally, 50 μL of Zika virus SZ-SMGC-01 strain (preserved by the Academy of Military Medical Sciences) diluted in DMEM medium containing 2% FBS was added to the cells, each well contained 100 $TCID_{50}$ of the virus. The final concentration of the compound to be tested was 0.25 times the pretreatment concentration, that was, the initial concentration was 200 μM, and dilution was carried out by 3-fold series, so that the final concentrations of the compound to be tested were: 200 μM, 66.67 μM, 22.22 μM, 7.41 μM, 2.47 μM, 0.82 μM, 0.27 μM, 0.09 μM. The final concentrations of the positive compound were: 20 μM, 6.67 μM, 2.22 μM, 0.74 μM, 0.25 μM, 0.082 μM, 0.027 μM, 0.009 μM. Negative control (to the cell well, DMSO and culture medium were added, without drug) and positive control (to the cell well, DMSO, culture medium and virus were added, without drug) were set. The cell culture plates were incubated at 37°C for 9 days.

③ The Buffer and the substrate of CellTiter-Glo® Chemiluminescent Cell Viability Assay (purchased from Promega, Cat. No. G7573) were mixed in the dark to prepare a working solution. The working solution was mixed with PBS (purchased from Gibco, Cat. No. 10010049) at a ratio of 4:6. After the medium in the cell culture plate was discarded, 100 μl of detection reagent was added to each well, and the 96-well plate was shaken for 5 min with an orbital shaker to induce cell lysis. After stabilizing the signal in the dark for 2 minutes, a microplate reader (purchased from Molecular Devices, model SpectraMax M5) was used to measure the chemiluminescent units, the plate reading program was the preset program of CellTiter-Glo, and the cell viability was calculated:

$$\text{Cell viability (\%)} = (A_{\text{(drug treatment group)}} - A_{\text{(positive control)}}) / (A_{\text{(negative control)}} - A_{\text{(positive control)}}) \times 100\%$$

wherein, A represented the reading of the microplate reader.

Result evaluation: origins.0 software was used to carry out the S-type curve fitting on the inhibition rate-concentration, and the $IC_{50}$ value of the compound to be tested was calculated.

(2) Anti-rhinovirus (HRV-1059) activity test

Test object: Rhinovirus (HRV-1059)-infected H1 Hela cells

Testing method:

① H1 Hela cells (purchased from the National Experimental Cell Resource Sharing Platform, Cat. No. 3111C0001CCC000344) were inoculated into a 96-well plate at a concentration of 1.5×10⁴ cells/well, and the cell culture medium was DMEM medium (purchased from Gibco, Cat. No. 11995065) containing 10% FBS (purchased

from Gibco, Cat. No. 16000044), the plate was was placed in a $CO_2$ incubator and incubated at 37°C for 24 hours.

② The original medium in the 96-well plate was discarded, 100 μL of DMEM medium (purchased from Gibco, Cat. No. 11995065) containing 2% FBS (purchased from Gibco, Cat. No. 16000044) was taken and added into the cells. Then the stock solution of the compound to be tested was serially diluted with the above DMEM medium to 800 μM, 266.67 μM, 88.89 μM, 29.63 μM, 9.88 μM, 3.29 μM, 1.10 μM, 0.37 μM, 0.12 μM, 0.04 μM, and 50 μL was added to the cell culture plate. The stock solution of positive compound NITD008 (purchased from MCE Company, Cat. No. HY-12957) was serially diluted with the above DMEM medium to 80 μM, 26.67 μM, 8.89 μM, 2.96 μM, 0.99 μM, 0.33 μM, 0.11 μM, 0.04 μM, 0.012 μM, 0.004 μM, 50 μL was added to the cell culture plate. Finally, 50 μL of the rhinovirus HRV-1059 strain (purchased from ATCC, Cat. No. VR-482) diluted with DMEM medium containing 2% FBS was added to the cells, the amount of virus contained in each well was 100 $TCID_{50}$. The final concentration of the compound to be tested was 0.25 times of the pretreatment concentration, that was, the initial concentration was 200 μM, the dilution was carried out by 3-fold series, the final concentrations of the compound to be tested were: 200 μM, 66.67 μM, 22.22 μM, 7.41 μM, 2.47 μM, 0.82 μM, 0.27 μM, 0.09 μM, 0.03 μM, 0.01 μM. The final concentrations of the positive compound were: 20 μM, 6.67 μM, 2.22 μM, 0.74 μM, 0.25 μM, 0.082 μM, 0.027 μM, 0.009 μM, 0.003 μM, 0.001 μM. Negative control (to the cell well, DMSO and medium were added, without drug) and positive control (to the cell well, DMSO, medium and virus were added, without drug) were set. The cell culture plates were incubated at 37°C for 6 days.

③ The Buffer and the substrate of CellTiter-Glo® Chemiluminescent Cell Viability Assay (purchased from Promega, Cat. No. G7573) was mixed in the dark to obtain a working solution. The working solution was mixed with PBS (purchased from Gibco, Cat. No. 10010049) at a ratio of 4:6. After the medium in the cell culture plate was discarded, 100 μl of detection reagent was added to each well, and the 96-well plate was shaken for 5 min with an orbital shaker to induce cell lysis. After stabilizing the signal in the dark for 2 minutes, a microplate reader (purchased from Molecular Devices, model SpectraMax M5) was used to measure the chemiluminescent units, the plate reading program was the preset program of CellTiter-Glo, and the cell viability was calculated:

$$\text{Cell viability (\%)} = (A_{\text{(drug treatment group)}} - A_{\text{(positive control)}}) / (A_{\text{(negative control)}} - A_{\text{(positive control)}}) \times 100\%$$

wherein, A represented the reading of the microplate reader.

Result evaluation: origin8.0 software was used to carry out the S-type curve fitting on the inhibition rate-concentration, and the $IC_{50}$ value of the compound to be tested was calculated.

Table 1: Antiviral activity results of representative nitrogen-containing derivatives of 3-deoxy-2-ketoaldonic acid

| Compound | Structure Formula | $IC_{50}$ (μM) | |
|---|---|---|---|
| | | Anti-ZIKV (BHK) | Anti-HRV (H1Hela) |
| NITD008 | | 1.00 | 0.05 |
| 4a | 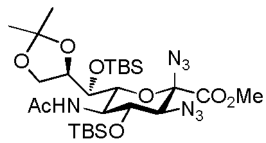 | 1.41 | 4.58 |
| 4b | 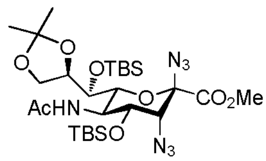 | 2.49 | 18.89 |
| 6a | 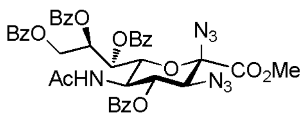 | 21.66 | >200 |

(continued)

| Compound | Structure Formula | $IC_{50}$ (μM) | |
|---|---|---|---|
| | | Anti-ZIKV (BHK) | Anti-HRV (H1Hela) |
| 6b | 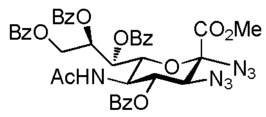 | 10.61 | >200 |
| 6c | 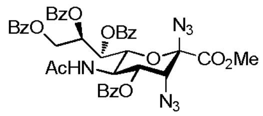 | 11.02 | >200 |
| 6d | 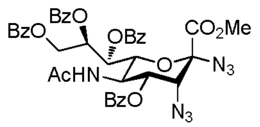 | 10.33 | >200 |
| 8a | 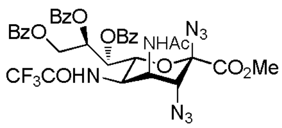 | 5.50 | 0.93 |
| 8b | 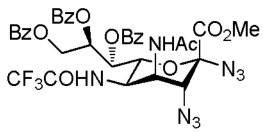 | 7.30 | 2.05 |
| 10a | 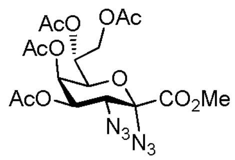 | >200 | 10.77 |
| 13 | 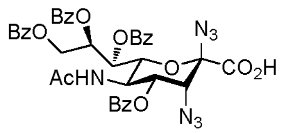 | 20.04 | 13.27 |
| 14 | 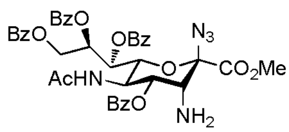 | 14.60 | >200 |
| 15 | 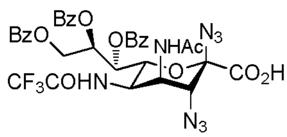 | 86.90 | 12.53 |
| 16 | 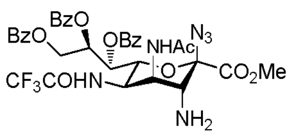 | 29.71 | 5.39 |

(continued)

| Compound | Structure Formula | $IC_{50}$ (μM) | |
|---|---|---|---|
| | | Anti-ZIKV (BHK) | Anti-HRV (H1Hela) |
| 17 | 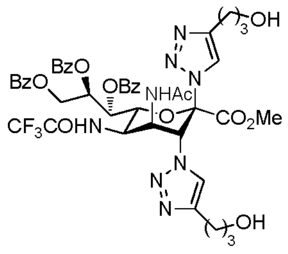 | 20.04 | >200 |
| 18 | 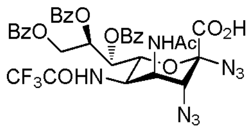 | 22.73 | 1.87 |
| 19 | 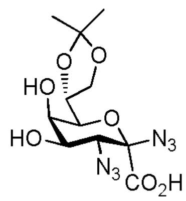 | >200 | 24.55 |

The above test results showed that the nitrogen-containing derivatives of 3-deoxy-2-ketoaldonic acid prepared in various examples of the present application showed moderate to strong antiviral activity against Zika virus (ZIKV) and rhinovirus (HRV).

Although the specific embodiments of the present application have been described in detail, those skilled in the art will understand that according to all the teachings disclosed, various modifications and substitutions can be made to those details, and these changes are all within the protection scope of the present application. The full scope of the present application is given by the appended claims and any equivalents thereof.

## Claims

1. A compound represented by general Formula I, a stereoisomer, a pharmaceutically acceptable salt, a pharmaceutically acceptable ester, a pharmaceutically acceptable hydrate or a pharmaceutically acceptable solvate thereof,

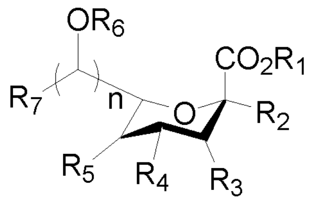

**I**

wherein:

$R_1$ is hydrogen, $C_1$-$C_6$ alkyl, allyl, phenyl or benzyl;
$R_2$ and $R_3$ are each independently azido, amino, amino substituted by one or two $R^a$, 5-membered or 6-membered nitrogen-containing heterocyclyl or 5-membered or 6-membered nitrogen-containing heterocyclyl substituted by $R^b$, wherein each $R^a$ is independently $C_1$-$C_6$ alkyl, allyl, phenyl, benzyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4;

$R_4$ and $R_5$ are each independently hydroxyl, amino, guanidino, hydroxyl substituted by $R^c$, amino substituted by $R^c$, guanidino substituted by $R^c$, 5-membered or 6-membered nitrogen-containing heterocyclyl or 5-membered or 6-membered nitrogen-containing heterocyclyl substituted by $R^b$, wherein each $R^c$ is independently $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl, methoxyformyl, tert-butoxyformyl or benzyloxyacyl, $R^b$ is $C_1$-$C_6$ alkyl, halogen or $-(CH_2)_mOH$, wherein m is 0, 1, 2, 3 or 4; or,
$R_4$ and $R_5$ together with the carbon atoms to which they connect form a 5-membered or 6-membered oxygen-containing or nitrogen-containing heterocyclic ring (e.g., 1,3-dioxolane, 1,3-dioxolan-2-one, dioxane, dioxanone, oxazolidine or oxazolidinone);
$R_6$ is hydrogen, $C_1$-$C_6$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, or trifluoroacetyl;
$R_7$ is hydrogen,

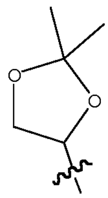
,

$R_6OCH_2$- or $R_6OCH_2(R_6O)CH$-, wherein $R_6$ is as defined above;
n is 0, 1, 2 or 3.

**2.** The compound, a stereoisomer, a pharmaceutically acceptable salt, a pharmaceutically acceptable ester, a pharmaceutically acceptable hydrate or a pharmaceutically acceptable solvate thereof according to claim 1, wherein:

preferably, $R_1$ is hydrogen or $C_1$-$C_4$ alkyl;
preferably, $R_2$ and $R_3$ are each independently azido, amino or 5-membered or 6-membered nitrogen-containing heterocyclyl substituted by $R^b$, wherein $R^b$ is $-(CH_2)_mOH$, wherein m is 0, 1, 2, 3 or 4, the 5-membered or 6-membered nitrogen-containing heterocyclyl is triazolyl, tetrazolyl, pyrrolyl, tetrahydropyrrolyl, piperidinyl, pyridyl, oxazolyl or imidazolyl;
preferably, $R_4$ and $R_5$ are each independently hydroxyl, amino, hydroxyl substituted by $R^c$, amino substituted by $R^c$, wherein each $R^c$ is independently $C_1$-$C_4$ alkyl, allyl, phenyl, benzyl, trimethylsilyl, triethylsilyl, triisopropylsilyl, tert-butyldimethylsilyl, tert-butyldiphenylsilyl, formyl, acetyl, benzoyl, trifluoroacetyl,_methoxyformyl, tert-butoxyformyl or benzyloxyacyl;
preferably, $R_4$ and $R_5$ together with the carbon atoms to which they connect form a 5-membered oxygen-containing heterocyclic ring (e.g., 1,3-dioxolane, 1,3-dioxolan-2-one);
preferably, $R_6$ is formyl, acetyl, benzoyl or trifluoroacetyl;
preferably, $R_7$ is

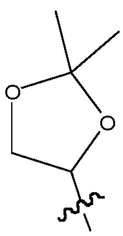
,

$R_6OCH_2$- or $R_6OCH_2(R_6O)CH$-, wherein $R_6$ is as defined above;
preferably, n is 0, 1 or 2.

**3.** The compound, a stereoisomer, a pharmaceutically acceptable salt, a pharmaceutically acceptable ester, a pharmaceutically acceptable hydrate or a pharmaceutically acceptable solvate thereof according to claim 1 or 2, wherein:

preferably, $R_1$ is hydrogen, methyl, ethyl, n-propyl, isopropyl, n-butyl, isobutyl, sec-butyl or tert-butyl;
preferably, $R_2$ and $R_3$ are each independently azido, amino or 5-membered nitrogen-containing heterocyclyl

substituted by $R^b$, wherein $R^b$ is - $(CH_2)_mOH$, wherein m is 0, 1, 2 or 3, the 5-membered nitrogen-containing heteroaryl is triazolyl, tetrazolyl, pyrrolyl, tetrahydropyrrolyl, oxazolyl or imidazolyl;
preferably, $R_4$ and $R_5$ are each independently hydroxyl, hydroxyl substituted by $R^c$, amino substituted by $R^c$, wherein each $R^c$ is independently methyl, ethyl, n-propyl, isopropyl, n-butyl, isobutyl, sec-butyl, tert-butyl, trimethylsilyl, triethylsilyl, triisopropylsilyl, tert-butyldimethylsilyl, formyl, acetyl, benzoyl, or trifluoroacetyl.

**4.** The compound, a stereoisomer, a pharmaceutically acceptable salt, a pharmaceutically acceptable ester, a pharmaceutically acceptable hydrate or a pharmaceutically acceptable solvent thereof according to any one of claims 1 to 3, wherein:

preferably, $R_1$ is hydrogen or methyl;
preferably, $R_2$ is azido or

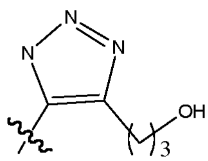
;

preferably, $R_3$ is azido, amino or

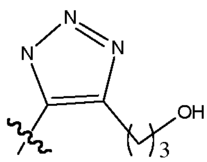
;

preferably, $R_4$ is hydroxyl, tert-butyldimethylsilyloxy, benzoyloxy, acetylamino or acetyloxy;
preferably, $R_5$ is hydroxyl, trifluoroacetyloxy, acetylamino or acetyloxy;
preferably, $R_4$ and $R_5$ together with the carbon atoms to which they connect form 1,3-dioxolane or 1,3-dioxolan-2-one;
preferably, $R_6$ is acetyl or benzoyl;
preferably, $R_7$ is

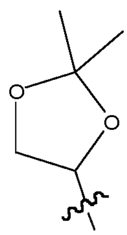

or $R_6OCH_2$, wherein $R_6$ is as defined above;
preferably, n is 0, 1 or 2.

**5.** The compound, a stereoisomer, a pharmaceutically acceptable salt, a pharmaceutically acceptable ester, a pharmaceutically acceptable hydrate or a pharmaceutically acceptable solvent thereof according to any one of claims 1 to 4, wherein the compound is selected from the group consisting of:

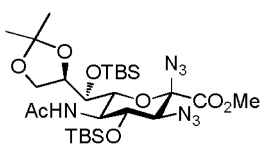
,
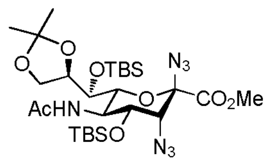
,
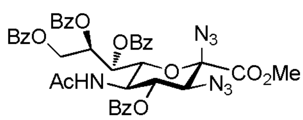
,

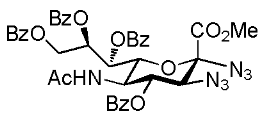,
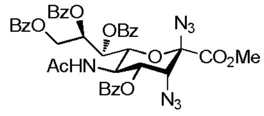,
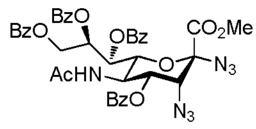,

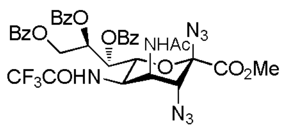,
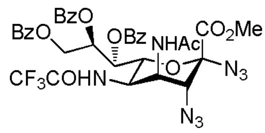,
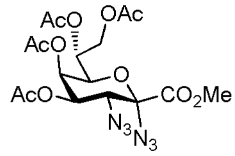,

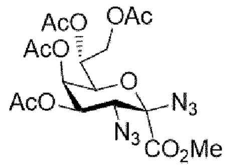,
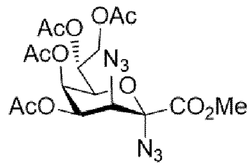,
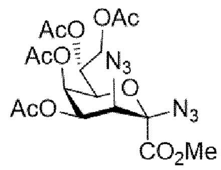,

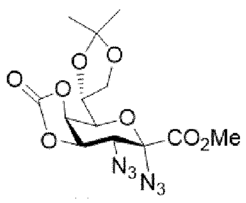,
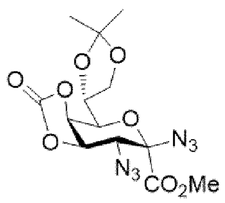,
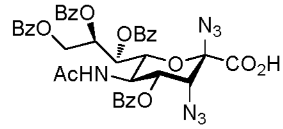,

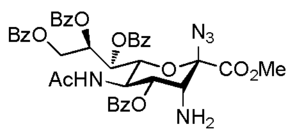,
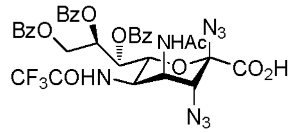,
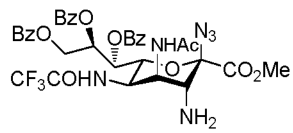,

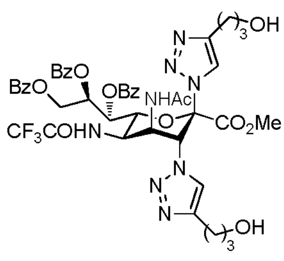,
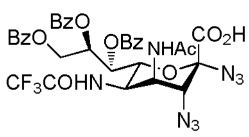, and
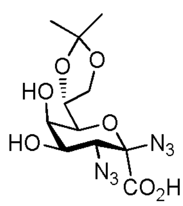.

6. A method for preparing the compound, a stereoisomer, a pharmaceutically acceptable salt, a pharmaceutically acceptable ester, a pharmaceutically acceptable hydrate or a pharmaceutically acceptable solvate thereof according to any one of claims 1 to 5, comprising:

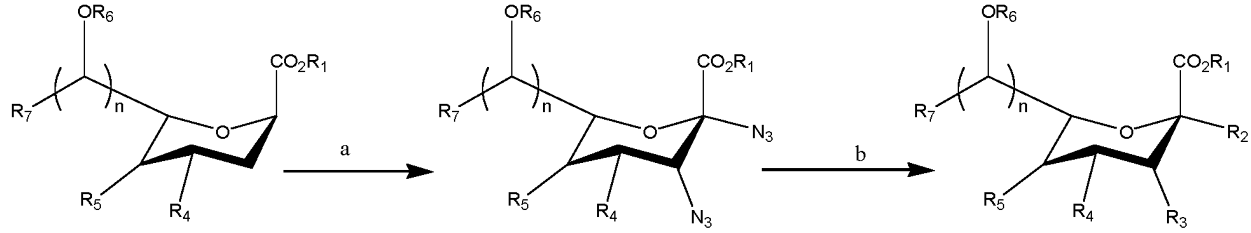


II III I

a) in a solvent, a compound represented by Formula II, a hypervalent iodine reagent and azidotrimethylsilane reacting under light conditions to generate a bis-azide represented by Formula III;
b) the bis-azide represented by Formula III generating the compound represented by the general Formula I through hydrolysis of the ester group, reduction of the azido group, removal of each protecting group on the hydroxyl or amino group, or formation of an azacycle,
wherein: the definition of $R_1$, $R_2$, $R_3$, $R_4$, $R_5$, $R_6$, $R_7$ is as described in any one of claims 1 to 5;
preferably, the hypervalent iodine reagent in step a) is selected from the group consisting of:

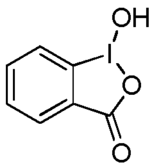, 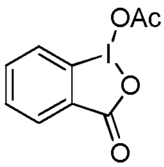, 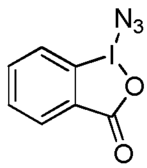, 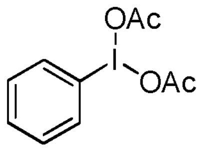, 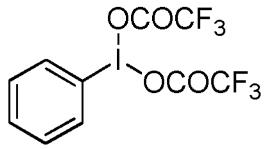,

and

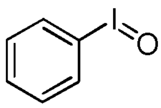,

etc., preferably is

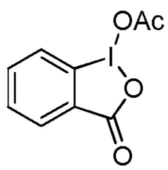;

preferably, the solvent in step a) is selected from the group consisting of dichloromethane, acetone, dimethyl sulfoxide, acetonitrile, etc., preferably is acetonitrile;
preferably, the light source for the light conditions in step a) is natural light or LED light of various colors, etc., preferably blue LED light;
preferably, the reaction in step a) is carried out at a temperature of 0-60°C, preferably under light conditions and at a temperature of 20-40°C;
preferably, the hydrolysis of the ester group in step b) is carried out in the presence of a base and a solvent, preferably, the base is selected from the group consisting of lithium hydroxide, sodium hydroxide, potassium hydroxide, sodium methoxide, sodium carbonate, potassium carbonate, sodium tert-butoxide, potassium tert-butoxide, and the solvent is water or alcoholic solvents, such as one or two selected from the group consisting of methanol, ethanol, isopropanol, and tert-butanol;
preferably, the reduction of the azido in step b) is carried out in the presence of a reducing agent and a solvent, preferably, the reducing agent is selected from the group consisting of triphenylphosphine, trimethylphosphine, tributylphosphine, palladium carbon/hydrogen, palladium hydroxide/hydrogen, and Raney nickel/hydrogen, and the solvent is water, tetrahydrofuran or alcoholic solvents, such as one or two selected from the group consisting of methanol, ethanol, isopropanol, and tert-butanol;
preferably, the removal of the protecting group in step b) is carried out in the presence of an acid or a base and a solvent, preferably, the acid is selected from the group consisting of hydrochloric acid, sulfuric acid, p-toluenesulfonic acid, and trifluoroacetic acid, the base is selected from the group consisting of sodium hydroxide, potassium hydroxide, sodium methoxide, sodium carbonate, potassium carbonate, sodium tert-butoxide, and potassium tert-butoxide, and the solvent is water, dichloromethane, tetrahydrofuran or alcohol solvents, such as one or two selected from the group consisting of methanol, ethanol, isopropanol, and tert-butanol;
preferably, the formation of azacycle in step b) is carried out in the presence of an alkyne, a copper catalyst and a solvent, preferably, the alkyne is selected from the group consisting of terminal alkynes or internal alkynes having various lengths with or without various functional groups, the copper catalyst is selected from the group consisting of cuprous chloride, cuprous bromide, cuprous iodide, and copper sulfate/sodium ascorbate, and the solvent is water, dichloromethane, tetrahydrofuran or alcoholic solvents, such as one or two selected from the

group consisting of methanol, ethanol, isopropanol, and tert-butanol.

7. A pharmaceutical composition comprising at least one the compound, a stereoisomer, a pharmaceutically acceptable salt, a pharmaceutically acceptable ester, a pharmaceutically acceptable hydrate or a pharmaceutically acceptable solvate thereof according to any one of claims 1 to 5, and one or more pharmaceutically acceptable carriers or excipients.

8. Use of the compound, a stereoisomer, a pharmaceutically acceptable salt, a pharmaceutically acceptable ester, a pharmaceutically acceptable hydrate or a pharmaceutically acceptable solvent thereof according to any one of claims 1 to 5, or the pharmaceutical composition according to claim 7 in the manufacture of a medicament for anti-virus (e.g., anti-Zika virus, anti-rhinovirus).

9. Use of the compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7 in the manufacture of a medicament for inhibiting the replication or reproduction of a virus (e.g., Zika virus, rhinovirus) in a cell (e.g., a cell of mammal).

10. Use of the compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7 in the manufacture of a medicament as an inhibitor against a virus (e.g., Zika virus, rhinovirus).

11. Use of the compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7 in the manufacture of a medicament for the prevention and/or treatment of a disease or infection caused by a virus (e.g., Zika virus, rhinovirus).

12. The compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7, for use as a medicament for anti-virus (e.g., anti-Zika virus, anti-rhinovirus).

13. The compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7, for use in inhibiting the replication or reproduction of a virus (e.g., Zika virus, rhinovirus) in a cell (e.g., a cell of mammal).

14. The compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7, for use as an inhibitor against a virus (e.g., Zika virus, rhinovirus).

15. The compound, a stereoisomer, a pharmaceutically acceptable salt, a pharmaceutically acceptable ester, a pharmaceutically acceptable hydrate or a pharmaceutically acceptable solvent thereof according to any one of claims 1 to 5 or the pharmaceutical composition according to claim 7, for use in the prevention and/or treatment of a disease or infection caused by a virus (e.g., Zika virus, rhinovirus).

16. A method for preventing and/or treating a disease or infection caused by a virus (e.g., Zika virus, rhinovirus) in a mammal in need, comprising administering to the mammal in need a therapeutically and/or prophylactically effective amount of the compound, a stereoisomer, a pharmaceutically acceptable salt, a pharmaceutically acceptable ester, a pharmaceutically acceptable hydrate or a pharmaceutically acceptable solvate thereof according to any one of claims 1 to 5 or the pharmaceutical composition according to claim 7.

17. A method for inhibiting the replication or reproduction of a virus (e.g., Zika virus, rhinovirus) in a mammal in need, comprising administering to the mammal in need a therapeutically and/or prophylactically effective amount of the compound, a stereoisomer, a pharmaceutically acceptable salt, a pharmaceutically acceptable ester, a pharmaceutically acceptable hydrate or a pharmaceutically acceptable solvate thereof according to any one of claims 1 to 5 or the pharmaceutical composition according to claim 7.

**18.** A method for inhibiting the replication or reproduction of a virus (e.g., Zika virus, rhinovirus) in a cell (e.g., a cell of mammal), comprising contacting the cell with the compound, a stereoisomer, a pharmaceutically acceptable salt, a pharmaceutically acceptable ester, a pharmaceutically acceptable hydrate or a pharmaceutically acceptable solvate thereof according to any one of claims 1 to 5 or the pharmaceutical composition according to claim 7.

**19.** The use according to claim 9, the compound, a stereoisomer, a pharmaceutically acceptable salt, a pharmaceutically acceptable ester, a pharmaceutically acceptable hydrate or a pharmaceutically acceptable solvate thereof or the pharmaceutical composition according to claim 13, or the method according to claims 16 to 18, wherein the mammal comprises bovine, equine, caprinae, suidae, canidae, feline, rodent, primate, among which the preferred mammal is a human, a cat, a dog or a pig.

**INTERNATIONAL SEARCH REPORT**

International application No. **PCT/CN2020/101906**

**A. CLASSIFICATION OF SUBJECT MATTER**

C07H 5/06(2006.01)i; C07H 1/00(2006.01)i; C12P 19/26(2006.01)i; C12P 19/44(2006.01)i; A61K 31/70(2006.01)i; A61P 31/00(2006.01)i

According to International Patent Classification (IPC) or to both national classification and IPC

**B. FIELDS SEARCHED**

Minimum documentation searched (classification system followed by classification symbols)

C07H 5/- C07H 1/- C12P 19/- A61K 31/- A61P 31/-

Documentation searched other than minimum documentation to the extent that such documents are included in the fields searched

Electronic data base consulted during the international search (name of data base and, where practicable, search terms used)

SIPOABS, CNTXT, CNPAT, DWPI, STN: 四川大学, 刘小宇, 去氧, 酮糖酸, 叠氮, 氨, 氮杂环, 病毒, carbohydrate, nitrogenous, antiviral, glycal diazidation

**C. DOCUMENTS CONSIDERED TO BE RELEVANT**

| Category* | Citation of document, with indication, where appropriate, of the relevant passages | Relevant to claim No. |
|---|---|---|
| A | KIM, Jin-Hyo et al. "Mechanism-Based Covalent Neuraminidase Inhibitors with Broad-Spectrum Influenza Antiviral Activity" *Science,* Vol. 340, No. 6128, 21 February 2013 (2013-02-21), pp. 71-75 | 1-19 |
| A | PASCOLUTTI, Mauro et al. "Structural Insights into Human Parainfluenza Virus 3 Hemagglutinin-Neuraminidase Using Unsaturated 3-N-Substituted Sialic Acids as Probes" *ACS Chemical Biology,* Vol. 13, No. 6, 25 August 2018 (2018-08-25), pp. 1544-1550 | 1-19 |
| A | CHENNAIAH, Ande et al. "Conversion of Glycals into Vicinal-1,2-Diazides and 1,2-(Or 2,1)-Azidoacetates Using Hypervalent Iodine Reagents and Me3SiN3. Application in the Synthesis of N-Glycopeptides, Pseudo-Trisaccharides and an Iminosugar" *RSC Advances,* Vol. 7, No. 66, 29 August 2017 (2017-08-29), pp. 41755-41762 | 1-19 |
| A | Meng, Xin et al. "Multivalent Neuraminidase Hydrolysis Resistant Triazole-Sialoside Protein Conjugates as Influenza-Adsorbents" *Chinese Chemical Letters,* Vol. 29, No. 1, 27 October 2017 (2017-10-27), pp. 76-80 | 1-19 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 March 2021** | **16 April 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

## INTERNATIONAL SEARCH REPORT

International application No.
**PCT/CN2020/101906**

**C. DOCUMENTS CONSIDERED TO BE RELEVANT**

| Category* | Citation of document, with indication, where appropriate, of the relevant passages | Relevant to claim No. |
|---|---|---|
| A | US 6664399 B1 (E. I. DU PONT DE NEMOURS AND COMPANY) 16 December 2003 (2003-12-16)<br>figure 4 | 1-19 |

**INTERNATIONAL SEARCH REPORT**

International application No.
**PCT/CN2020/101906**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **16-19**
because they relate to parts of the international application that do not comply with the prescribed requirements to such an extent that no meaningful international search can be carried out, specifically:

[1] Claims 16-19 relate to methods of treatment of diseases, and this search report is made on the basis of the use of a compound, a stereoisomer thereof, a pharmaceutically acceptable salt thereof, a pharmaceutically acceptable ester thereof, a pharmaceutically acceptable hydrate thereof, a pharmaceutically acceptable solvent compound thereof, or a pharmaceutical composition in the preparation of a drug for the treatment of a disease.

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/101906**

| Patent document cited in search report | Publication date (day/month/year) | Patent family member(s) | Publication date (day/month/year) |
|---|---|---|---|
| US 6664399 B1 | 16 December 2003 | US 2005124563 A1 | 09 June 2005 |
| | | US 2004059105 A1 | 25 March 2004 |
| | | US 6852866 B2 | 08 February 2005 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Science,* 2013, vol. 340, 71-75
- *ACS Chem. Biol.,* 2018, vol. 13, 1544-1550